# EUROPEAN PATENT SPECIFICATION

(11) **EP 4 268 709 B1**
(45) Date of publication and mention of the grant of the patent: **01.10.2025**
(21) Application number: 22745239.8
(22) Date of filing: 25.01.2022
(51) Int. Cl.: A61B 5/346, A61B 5/00, A61B 5/349

(54) **METHOD AND APPARATUS FOR DETERMINING PROBABILITY OF SUFFERING FROM CORONARY HEART DISEASE**
VERFAHREN UND VORRICHTUNG ZUR BESTIMMUNG DER WAHRSCHEINLICHKEIT DES LEIDENS AN KORONARER HERZERKRANKUNG
PROCÉDÉ ET APPAREIL POUR DÉTERMINER LA PROBABILITÉ DE SOUFFRIR DE CORONAROPATHIE

(30) Priority: 30.01.2021 CN 202110131912
(43) Date of publication of application: 01.11.2023
(73) Proprietor: Huawei Technologies Co., Ltd., Shenzhen, Guangdong 518129 (CN)
(72) Inventor: SI, Xiaoyun, Shenzhen, Guangdong 518129 (CN)
(74) Representative: Epping - Hermann - Fischer
(86) International application number: PCT/CN2022/073854
(87) International publication number: WO 2022/161365

(56) References cited:
- CN-A- 107 714 023
- CN-A- 110 090 012
- CN-A- 110 090 012
- CN-A- 111 652 290
- US-A1- 2008 114 259
- US-A1- 2013 172 763
- US-A1- 2016 022 164
- US-A1- 2020 205 745
- US-A1- 2020 211 713

## Description

### CROSS-REFERENCE TO RELATED APPLICATIONS

This application claims priority to Chinese Patent Application No. 202110131912.2, filed with the China National Intellectual Property Administration on January 30, 2021 and entitled "METHOD AND APPARATUS FOR DETERMINING CORONARY HEART DISEASE PROBABILITY".

### TECHNICAL FIELD

Embodiments of this application relate to the field of terminal technologies, and in particular, to a method and an apparatus for determining a coronary heart disease probability.

### BACKGROUND

Coronary heart disease refers to coronary atherosclerosis heart disease, which is caused by myocardial ischemia and hypoxia resulting from coronary stenosis and obstruction due to atherosclerosis. According to a pathology of coronary heart disease, a most effective diagnostic evidence of coronary heart disease is discovery of an intravascular physical change. Therefore, a gold standard for coronary heart disease detection is an invasive detection technology, like coronary angiography. Coronary angiography refers to a radioimaging examination technology of an anatomic morphology of a coronary artery by injecting a contrast agent through a special coronary arteriography catheter. However, coronary angiography is invasive, expensive, and complex in operation.

In addition, the following methods may be further used to assist in diagnosis of coronary heart disease. For example, biomarkers of troponin and myoglobin in a blood test can also be used for acute coronary heart disease detection. For another example, an ability of a coronary artery to supply blood and oxygen to a heart may alternatively be indirectly evaluated based on a supply and demand level of oxygen consumption of a patient's heart, to indirectly detect coronary heart disease. For example, coronary heart disease can be indirectly detected by performing electrocardiogram signal analysis and a load test. However, due to specific limitations, all of these methods are recommended only to assist in diagnosis of coronary heart disease, with poor convenience.

Therefore, a method and an apparatus that can conveniently estimate a coronary heart disease probability are urgently needed, to further remind a user to seek medical treatment or remind a doctor to perform further examinations such as a coronary angiography examination for the user. CN 110 090 012 A relates to a human body diseases detection method and testing product based on machine learning, which include: extract electrocardial vector data in data characteristics and its quantizating index data, construct the machine learning classification model of electrocardial vector data characteristics, and respective weights value is assigned to the different classifications result of model identification, obtain the comprehensive judgement result of human body diseases detection.

US 2020/205745 A1 relates to a method comprising receiving, by a processor, a biophysical signal data set of a subject acquired from one or more channels of one or more sensors; pre-processing the biophysical signal data set to generate one or more pre-processed data sets, wherein each preprocessed data set includes a single isolated complete cardiac cycle; and determining, by the processor, a value indicative of presence of cardiac disease or condition by directly inputting the pre-processed data set to one or more deep neural networks trained with a set of training biophysical signal data set acquired from patients diagnosed with the cardiac disease or condition and labeled with the presence or non-presence of the cardiac disease or condition, wherein an output data set is outputted via a report and/or a display based on the determined value indicative of the presence of cardiac disease or condition.

CN 111 652 290 A relates to a method and a device for detecting a confrontation sample, wherein the method comprises the following steps: generating pixel characteristic data of a picture to be detected according to pixel information of the picture to be detected and a preset format, and using the pixel characteristic data as a sample to be detected; inputting a sample to be detected into a specific sample reconstruction model to obtain a reconstructed sample of the sample to be detected; inputting the reconstructed sample of the sample to be detected into the specific pre-estimation model to obtain an intermediate layer output value of the reconstructed sample of the sample to be detected; and inputting the intermediate layer output value of the reconstructed sample of the sample to be detected into a specific classification model, and determining whether the sample to be detected is a countermeasure sample.

### SUMMARY

Embodiments of this application provide a method and an apparatus for determining a coronary heart disease probability, to estimate a coronary heart disease probability. The invention is set out in the appended claims.

According to a first aspect, an embodiment of this application provides a method for determining a coronary heart disease probability. The method includes: obtaining pre-obtained ECG signals of one or more leads of a user; determining, based on the ECG signals of the one or more leads, reconstructed signals respectively corresponding to the ECG signals of the one or more leads; and determining a coronary heart disease probability of the user based on the ECG signals of the one or more leads and the reconstructed signals respectively corresponding to the ECG signals of the one or more leads. The ECG signals of the one or more leads include an ECG signal of a first lead, and a reconstructed signal corresponding to the ECG signal of the first lead is obtained by performing signal reconstruction based on a feature of the ECG signal of the first lead. According to the foregoing method, a coronary heart disease probability may be estimated.

To determine the coronary heart disease probability of the user based on the ECG signals of the one or more leads and the reconstructed signals respectively corresponding to the ECG signals of the one or more leads, the following method is used: determining an abnormal signal in the ECG signals of the one or more leads based on the ECG signals of the one or more leads and the reconstructed signals respectively corresponding to the ECG signals of the one or more leads; and determining the coronary heart disease probability of the user based on the abnormal signal and a corresponding reconstructed signal.

Accordingly, an abnormal signal is first determined, and then a coronary heart disease probability of a user is determined based on an abnormal signal and a corresponding reconstructed signal.

When the reconstructed signals respectively corresponding to the ECG signals of the one or more leads are determined based on the ECG signals of the one or more leads, a feature of an ECG signal of each lead in the ECG signals of the one or more leads is extracted by using a first model; and signal reconstruction is performed based on the feature of the ECG signal of each lead by using a second model, to obtain a corresponding reconstructed signal. The first model and the second model are obtained through training based on an ECG signal of each lead of a healthy population. Accordingly, the reconstructed signal corresponding to the ECG signal of each lead in the ECG signals of the one or more leads is determined by using the first model and the second model. In addition, the first model and the second model may not be distinguished, but are combined into one model, to obtain, from the ECG signals of the one or more leads, the reconstructed signal corresponding to the ECG signal of each lead.

In a possible design, when the abnormal signal in the ECG signals of the one or more leads is determined based on the ECG signals of the one or more leads and the reconstructed signals respectively corresponding to the ECG signals of the one or more leads, it is determined that the ECG signal of the first lead is an abnormal signal if the ECG signal of the first lead and the corresponding reconstructed signal meet one or more of the following abnormal signal conditions: In differences between the ECG signal of the first lead and the corresponding reconstructed signal, an absolute value of a difference is greater than a first threshold. Alternatively, in differences between the ECG signal of the first lead and the corresponding reconstructed signal, a ratio of an absolute value of a difference to an unsigned greatest value is greater than a second threshold, where the unsigned greatest value is a greatest value in absolute values of amplitudes of the ECG signal of the first lead. Alternatively, a sum of squares of absolute values of differences between the ECG signal of the first lead and the corresponding reconstructed signal is greater than a third threshold. Alternatively, a sum of squares of ratios of absolute values of differences between the ECG signal of the first lead and the corresponding reconstructed signals to an unsigned greatest value is greater than a fourth threshold, where the unsigned greatest value is a greatest value in absolute values of amplitudes of the ECG signal of the first lead. Alternatively, a standard deviation of differences between the ECG signal of the first lead and the corresponding reconstructed signal is greater than a fifth threshold. Alternatively, a standard deviation of ratios of differences between the ECG signal of the first lead and the corresponding reconstructed signal to an unsigned greatest value is greater than a sixth threshold, where the unsigned greatest value is a greatest value in absolute values of amplitudes of the ECG signal of the first lead. Alternatively, an X1 percentile of absolute values of differences between the ECG signal of the first lead and the corresponding reconstructed signal is greater than a seventh threshold, where X1 is a preset value. Alternatively, an X2 percentile of ratios of absolute values of differences between the ECG signal of the first lead and the corresponding reconstructed signal to an unsigned greatest value is greater than an eighth threshold. X2 is a preset value, and the unsigned greatest value is a greatest value in absolute values of amplitudes of the ECG signal of the first lead.

According to the foregoing design, the abnormal signal in the ECG signals of the one or more leads may be determined.

In a possible design, when the coronary heart disease probability of the user is determined based on the abnormal signal and the corresponding reconstructed signal, a target signal is determined based on the abnormal signal and the corresponding reconstructed signal, where the target signal reflects a difference between the abnormal signal and the corresponding reconstructed signal. The coronary heart disease probability of the user is determined based on the target signal by using a third model. The third model is obtained through training based on a first signal set and a second signal set. The first signal set includes target signals respectively corresponding to ECG signals of leads of a patient having coronary heart disease, and the target signals respectively corresponding to the ECG signals of the leads of the patient having coronary heart disease are determined by the ECG signals of the leads of the patient having coronary heart disease and reconstructed signals respectively corresponding to the ECG signals of the leads of the patient having coronary heart disease. The second signal set includes target signals respectively corresponding to ECG signals of the leads of a healthy population, and the target signals respectively corresponding to the ECG signals of the leads of the healthy population are determined by the ECG signals of the leads of the healthy population and reconstructed signals respectively corresponding to the ECG signals of the leads of the healthy population. For example, a sum of a coronary heart disease probability of the user and a probability that the user belongs to a healthy group is 1.

In a possible design, when the coronary heart disease probability of the user is determined based on the abnormal signal and the corresponding reconstructed signal, a target signal is determined based on the abnormal signal and the corresponding reconstructed signal, where the target signal reflects a difference between the abnormal signal and the corresponding reconstructed signal. The coronary heart disease probability of the user is determined based on the target signal by using a third model. The third model is obtained through training based on a first signal set, a second signal set, and a third signal set. The first signal set includes target signals respectively corresponding to ECG signals of leads of a patient having coronary heart disease, and the target signals respectively corresponding to the ECG signals of the leads of the patient having coronary heart disease are determined by the ECG signals of the leads of the patient having coronary heart disease and reconstructed signals respectively corresponding to the ECG signals of the leads of the patient having coronary heart disease. The second signal set includes target signals respectively corresponding to ECG signals of leads of a healthy population, and the target signals respectively corresponding to the ECG signals of the leads of the healthy population are determined by the ECG signals of the leads of the healthy population and reconstructed signals respectively corresponding to the ECG signals of the leads of the healthy population. The third signal set includes target signals respectively corresponding to ECG signals of leads of a population having another disease, and the target signals respectively corresponding to the ECG signals of the leads of the population having another disease are determined by the ECG signals of the leads of the population having another disease and reconstructed signals respectively corresponding to the ECG signals of the leads of the population having another disease. For example, a sum of a coronary heart disease probability of the user, an another disease probability of the user, and a probability that the user belongs to a healthy population is 1.

According to the foregoing design, a coronary heart disease probability of a user may be determined based on an abnormal signal and a corresponding reconstructed signal.

**In** a possible design, the following is further included: determining a first feature based on the abnormal signal and the corresponding reconstructed signal; determining location information of the first feature in coronary heart disease lesion space based on the first feature by using the third model; and determining a most probable coronary heart disease lesion location of the user based on the location information of the first feature in the coronary heart disease lesion space. According to the foregoing design, a most probable coronary heart disease lesion location of a user can be determined.

In a possible design, the coronary heart disease lesion space is obtained through training based on the first signal set and diagnostic labels of the ECG signals of the leads of the patient having coronary heart disease.

In a possible design, the first feature is a fully-connected layer feature.

According to a second aspect, an embodiment of this application provides an apparatus for determining a coronary heart disease probability. The apparatus includes a transceiver unit and a processing unit. The transceiver unit is configured to obtain electrocardiogram ECG signals of one or more leads of a user. The processing unit is configured to: determine, based on the ECG signals of the one or more leads, reconstructed signals respectively corresponding to the ECG signals of the one or more leads, where the ECG signals of the one or more leads include an ECG signal of a first lead, and a reconstructed signal corresponding to the ECG signal of the first lead is obtained by performing signal reconstruction based on a feature of the ECG signal of the first lead; and determine a coronary heart disease probability of the user based on the ECG signals of the one or more leads and the reconstructed signals respectively corresponding to the ECG signals of the one or more leads.

The processing unit is configured to: when the coronary heart disease probability of the user is determined based on the ECG signals of the one or more leads and the reconstructed signals respectively corresponding to the ECG signals of the one or more leads, determine an abnormal signal in the ECG signals of the one or more leads based on the ECG signals of the one or more leads and the reconstructed signals respectively corresponding to the ECG signals of the one or more leads; and determine the coronary heart disease probability of the user based on the abnormal signal and a corresponding reconstructed signal.

The processing unit is configured to: when the reconstructed signals respectively corresponding to the ECG signals of the one or more leads are determined based on the ECG signals of the one or more leads, extract a feature of an ECG signal of each lead in the ECG signals of the one or more leads by using a first model; and perform signal reconstruction based on the feature of the ECG signal of each lead by using a second model, to obtain a reconstructed signal corresponding to the ECG signal of each lead. The first model and the second model are obtained through training based on an ECG signal of each lead of a healthy population.

In a possible design, the processing unit is configured to: when the abnormal signal in the ECG signals of the one or more leads is determined based on the ECG signals of the one or more leads and the reconstructed signals respectively corresponding to the ECG signals of the one or more leads, determine that the ECG signal of the first lead is an abnormal signal if the ECG signal of the first lead and the corresponding reconstructed signal meet one or more of the following abnormal signal conditions: In differences between the ECG signal of the first lead and the corresponding reconstructed signal, an absolute value of a difference is greater than a first threshold. Alternatively, in differences between the ECG signal of the first lead and the corresponding reconstructed signal, a ratio of an absolute value of a difference to an unsigned greatest value is greater than a second threshold, where the unsigned greatest value is a greatest value in absolute values of amplitudes of the ECG signal of the first lead. Alternatively, a sum of squares of absolute values of differences between the ECG signal of the first lead and the corresponding reconstructed signal is greater than a third threshold. Alternatively, a sum of squares of ratios of absolute values of differences between the ECG signal of the first lead and the corresponding reconstructed signal to an unsigned greatest value is greater than a fourth threshold, where the unsigned greatest value is a greatest value in absolute values of amplitudes of the ECG signal of the first lead. Alternatively, a standard deviation of differences between the ECG signal of the first lead and the corresponding reconstructed signal is greater than a fifth threshold. Alternatively, a standard deviation of ratios of differences between the ECG signal of the first lead and the corresponding reconstructed signal to an unsigned greatest value is greater than a sixth threshold, where the unsigned greatest value is a greatest value in absolute values of amplitudes of the ECG signal of the first lead. Alternatively, an X1 percentile of absolute values of differences between the ECG signal of the first lead and the corresponding reconstructed signal is greater than a seventh threshold, where X1 is a preset value. Alternatively, an X2 percentile of ratios of absolute values of differences between the ECG signal of the first lead and the corresponding reconstructed signal to an unsigned greatest value is greater than an eighth threshold. X2 is a preset value, and the unsigned greatest value is a greatest value in absolute values of amplitudes of the ECG signal of the first lead.

In a possible design, the processing unit is configured to: when the coronary heart disease probability of the user is determined based on the abnormal signal and the corresponding reconstructed signal, determine a target signal based on the abnormal signal and the corresponding reconstructed signal, where the target signal reflects a difference between the abnormal signal and the corresponding reconstructed signal; and determine the coronary heart disease probability of the user based on the target signal by using a third model.

The third model is obtained through training based on a first signal set and a second signal set. The first signal set includes target signals respectively corresponding to ECG signals of leads of a patient having coronary heart disease, and the target signals respectively corresponding to the ECG signals of the leads of the patient having coronary heart disease are determined by the ECG signals of the leads of the patient having coronary heart disease and reconstructed signals respectively corresponding to the ECG signals of the leads of the patient having coronary heart disease. The second signal set includes target signals respectively corresponding to ECG signals of the leads of a healthy population, and the target signals respectively corresponding to the ECG signals of the leads of the healthy population are determined by the ECG signals of the leads of the healthy population and reconstructed signals respectively corresponding to the ECG signals of the leads of the healthy population.

In a possible design, the processing unit is configured to: when the coronary heart disease probability of the user is determined based on the abnormal signal and the corresponding reconstructed signal, determine a target signal based on the abnormal signal and the corresponding reconstructed signal, where the target signal reflects a difference between the abnormal signal and the corresponding reconstructed signal; and determine the coronary heart disease probability of the user based on the target signal by using a third model.

The third model is obtained through training based on a first signal set, a second signal set, and a third signal set. The first signal set includes target signals respectively corresponding to ECG signals of leads of a patient having coronary heart disease, and the target signals respectively corresponding to the ECG signals of the leads of the patient having coronary heart disease are determined by the ECG signals of the leads of the patient having coronary heart disease and reconstructed signals respectively corresponding to the ECG signals of the leads of the patient having coronary heart disease. The second signal set includes target signals respectively corresponding to ECG signals of leads of a healthy population, and the target signals respectively corresponding to the ECG signals of the leads of the healthy population are determined by the ECG signals of the leads of the healthy population and reconstructed signals respectively corresponding to the ECG signals of the leads of the healthy population. The third signal set includes target signals respectively corresponding to ECG signals of leads of a population having another disease, and the target signals respectively corresponding to the ECG signals of the leads of the population having another disease are determined by the ECG signals of the leads of the population having another disease and reconstructed signals respectively corresponding to the ECG signals of the leads of the population having another disease.

In a possible design, the processing unit is configured to: determine a first feature based on the abnormal signal and the corresponding reconstructed signal by using the third model; determine location information of the first feature in coronary heart disease lesion space based on the first feature; and determine a most probable coronary heart disease lesion location of the user based on the location information of the first feature in the coronary heart disease lesion space.

In a possible design, the coronary heart disease lesion space is obtained through training based on the first signal set and diagnostic labels of the ECG signals of the leads of the patient having coronary heart disease.

In a possible design, the first feature is a fully-connected layer feature.

According to a third aspect, an embodiment of this application provides a computer-readable storage medium. The storage medium stores a computer program or instructions. When the computer program is executed or the instructions are executed by a communication apparatus, any one of the first aspect and the possible designs of the first aspect is implemented.

### BRIEF DESCRIPTION OF DRAWINGS

FIG. 1 shows schematic diagrams of various possible features of an ST segment according to an embodiment of this application;
FIG. 2 shows schematic diagrams of various possible features of a T wave according to an embodiment of this application;
FIG. 3 shows schematic diagrams of feature extraction and reconstruction of ECG signals of leads of a healthy population according to an embodiment of this application;
FIG. 4 shows schematic diagrams of ECG signals of leads of a healthy population and reconstructed signals corresponding to the ECG signals of the leads of the healthy population according to an embodiment of this application;
FIG. 5 shows schematic diagrams of ECG signals of leads of a patient having coronary heart disease and reconstructed signals corresponding to the ECG signals of the leads of the patient having coronary heart disease according to an embodiment of this application;
FIG. 6 is a schematic diagram of coronary heart disease lesion space according to an embodiment of this application;
FIG. 7 is an overview flowchart of determining a coronary heart disease probability according to an embodiment of this application;
FIG. 8 is a schematic diagram of coronary heart disease lesion space corresponding to fully-connected layer features according to an embodiment of this application;
FIG. 9A is a schematic diagram of a posture and a method for collecting an ECG signal by an ECG wearable watch according to an embodiment of this application;
FIG. 9B is a schematic diagram 1 of ECG signals of six leads according to an embodiment of this application;
FIG. 9C is a schematic diagram 1 of reconstructed signals corresponding to ECG signals of six leads according to an embodiment of this application;
FIG. 9D is a schematic diagram 1 of leads displaying abnormal signals according to an embodiment of this application;
FIG. 9E is a schematic diagram of displaying coronary heart disease lesion space of a user A according to an embodiment of this application;
FIG. 9F is a schematic diagram of displaying a coronary heart disease probability of a user A according to an embodiment of this application;
FIG. 10A is a schematic diagram of contact parts at which a heart rate chest strap collects ECG signals according to an embodiment of this application;
FIG. 10B is a schematic diagram 2 of ECG signals of six leads according to an embodiment of this application;
FIG. 10C is a schematic diagram 2 of reconstructed signals corresponding to ECG signals of six leads according to an embodiment of this application;
FIG. 10D is a schematic diagram 2 of leads displaying abnormal signals according to an embodiment of this application;
FIG. 10E is a schematic diagram of displaying coronary heart disease lesion space of a user B according to an embodiment of this application;
FIG. 10F is a schematic diagram of displaying a coronary heart disease probability of a user B according to an embodiment of this application;
FIG. 11A is a schematic diagram of ECG signals of twelve leads according to an embodiment of this application;
FIG. 11B is a schematic diagram of reconstructed signals corresponding to ECG signals of twelve leads according to an embodiment of this application;
FIG. 11C is a schematic diagram of leads displaying abnormal signals in ECG signals of twelve leads according to an embodiment of this application;
FIG. 11D is a schematic diagram of displaying coronary heart disease lesion space of a user C according to an embodiment of this application;
FIG. 11E is a schematic diagram of displaying a coronary heart disease probability of a user C according to an embodiment of this application;
FIG. 12 is a schematic diagram 1 of a structure of a communication apparatus according to an embodiment of this application; and
FIG. 13 is a schematic diagram 2 of a structure of a communication apparatus according to an embodiment of this application.

### DESCRIPTION OF EMBODIMENTS

In a broad sense, etiologies of coronary heart disease include inflammation, spasm, embolization, trauma, and congenital malformation of coronary artery in addition to coronary atherosclerosis. However, a non-coronary sclerosis etiology is very rare. According to symptoms, coronary heart disease can be divided into a hidden type, an angina type, a myocardial infarction type, a heart failure type, and a sudden death type.

An electrocardiogram vector generated during cardiac depolarization and repolarization is transmitted to each part of a body through volume conduction, and a potential difference is generated. Two electrodes are placed at any two points of a human body and connected to an electrocardiograph, so that an electrocardiogram can be recorded. A line on which electrodes are placed and connected to an electrocardiograph is called an electrocardiogram lead. A commonly used lead is a standard lead. The standard lead is also known as a bipolar limb lead, and reflects a potential difference between two limbs. An international universal lead system is used. In the system, there are twelve leads in total, that is, I, II, III, avL, avF, avR, v1, v2, v3, v4, v5, and v6. For example, an I lead is a lead that connects an electrode on a left upper limb to a positive end of an electrocardiograph, and connects an electrode on a right upper limb to a negative end, to reflect a potential difference between the left upper limb (l) and the right upper limb (r). When a potential of l is higher than a potential of r, an upward waveform is recorded; or when a potential of r is higher than a potential of l, a downward waveform is recorded. An II lead is a lead that connects an electrode on a left lower limb to a positive end of an electrocardiograph, and connects an electrode on a right upper limb to a negative end, to reflect a potential difference between the left lower limb (F) and the right upper limb (r). When a potential of f is higher than a potential of r, an upward wave is recorded; or when a potential of r is higher than a potential of f, a downward wave is recorded. An III lead is a lead that connects an electrode on a left lower limb to a positive end of an electrocardiograph, and connects an electrode on a left upper limb to a negative end, to reflect a potential difference between the left lower limb (F) and the left upper limb (l). When a potential of f is higher than a potential of l, an upward wave is recorded; or when a potential of l is higher than a potential of f, a downward wave is recorded.

Generally, there are mainly four typical electrocardiogram manifestations of coronary heart disease. (1) A T wave is bidirectional, low flat or inverted. (2) An ST segment is moved downward or elevated. (3) An R wave progressively decreases or disappears. (4) An electrocardiogram changes dynamically. If there are typical clinical symptoms of coronary heart disease and there are characteristic changes in an electrocardiogram, in most of these cases, coronary heart disease can be directly diagnosed through electrocardiograms, except for cases of a small population like menopause women. In addition, if there are typical clinical symptoms of coronary heart disease but there is no significant change in a common electrocardiogram, a possible cause is that an arterial circulation of a heart has a large blood supply capacity and it is difficult to detect an abnormality during resting. In this case, an exercise plate electrocardiogram test is needed to find a real change in an electrocardiogram. A coronary angiography examination is further needed if necessary. Therefore, an electrocardiogram is an indispensable and optimal examination method in diagnosis of coronary heart disease, and provides important clinical reference value for diagnosis of coronary heart disease. Main advantages of using an electrocardiogram in diagnosis of myocardial infarction include: (1) being conducive to early diagnosis and intervention of myocardial infarction because electrocardiogram changes occur earlier; (2) being not only qualitative, but also positioning; (3) being not only diagnostic, but also staging; (4) having prognosis judgment value; (5) being simple in equipment, popular, non-invasive in inspection, convenient, repeatable, economical, and the like.

A fundamental pathology of coronary heart disease is that coronary artery blood supply is poor, insufficient, ischemic, or the like. Consequently, ST-T changes are shown on an electrocardiogram. Typical manifestations are a horizontally downward ST segment, a disappeared T wave or an inverted T wave, and the like. The following describes various abnormal features that may be included in an electrocardiogram.
1. ST-segment depression: A normal ST segment is usually smoothly connected to a T wave. Therefore, frequently, it is difficult to determine an end of an ST segment and a start of a T wave. Typical myocardial ischemia causes ST-segment depression. An earliest and most subtle change in the ST segment is flattening of the ST segment. This causes a more obvious angle between the ST segment and a T wave.
2. ST-segment elevation: Transient ST-segment elevation in patients having chest pain is a feature of myocardial ischemia, and is usually found in coronary spastic angina. Myocardial ischemia in some of the patients develop into severe proximal coronary stenosis. When ST-segment elevation occurs and then subsides, deep T-wave inversion may occur subsequently (even without evidence of a myocardial injury enzymatic abnormality).

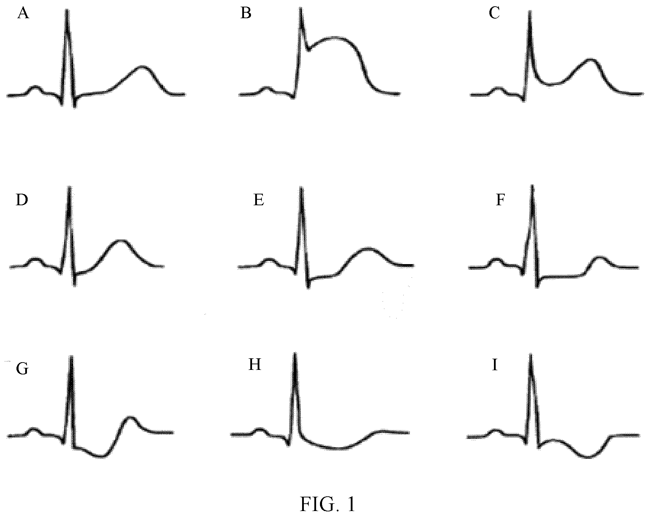

FIG. 1 shows schematic diagrams A to I of various possible features of an ST segment. A shows a schematic diagram of a normal ST segment; B shows a schematic diagram of an ST segment whose bow back is elevated upward; C shows a schematic diagram of an ST segment whose bow back is elevated downward; D shows a schematic diagram of an ST segment in quickly up-sloping depression; E shows a schematic diagram of an ST segment in slowly up-sloping depression; F shows a schematic diagram of an ST segment in horizontal depression; G shows a schematic diagram of an ST segment in quickly down-sloping depression; and H shows a schematic diagram of an ST segment in slowly down-sloping depression; and I shows a schematic diagram of an ST segment in fish-hook pattern depression.

3. T-wave change: Myocardial ischemia may cause various T-wave changes, such as a T-wave peak, a T-wave biphasic change, T-wave inversion, or T-wave flatness.

Deep and symmetric T-wave inversion strongly suggests myocardial ischemia. However, T-wave inversion may alternatively be a normal phenomenon. For example, in III, aVR, and V1 leads, T-wave inversion with a downward main wave of a QRS wave is normal.

For the T-wave biphasic change, a biphasic T wave is manifested in some patients having nonpermeable myocardial ischemia. Especially, a T-wave biphasic change occurring in an anterior thoracic lead is a feature of acute myocardial ischemia. A biphasic T wave usually progresses and evolves into a symmetric T wave. These changes occur in a patient having unstable angina or variant angina, and strongly suggest myocardial ischemia.

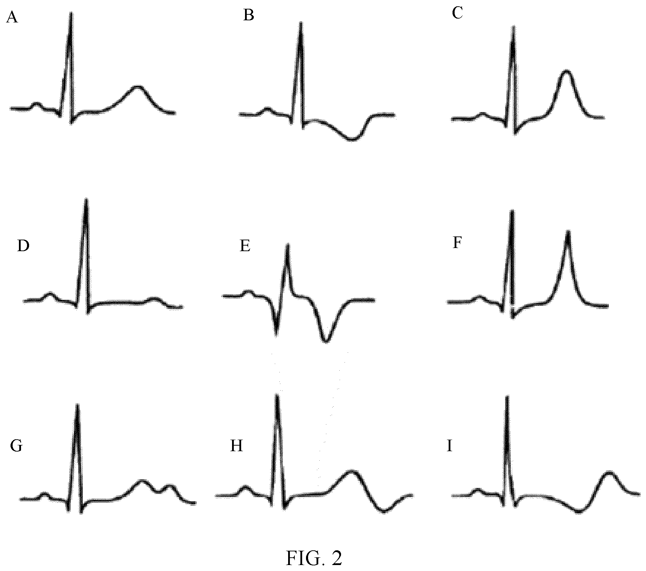

FIG. 2 shows schematic diagrams A to I of various possible features of a T wave. A shows a schematic diagram of a normal T wave; B shows a schematic diagram of an inverted T wave; C shows a schematic diagram of a tall upright T wave; D shows a schematic diagram of a low flat T wave; E shows a schematic diagram of a coronary T wave; F shows a schematic diagram of a peaked T wave; G shows a schematic diagram of a double-peaked T wave; H shows a schematic diagram of a biphasic T wave that is positive first and then negative; and I shows a schematic diagram of a biphasic T wave that is negative first and then positive.

It can be learned from the foregoing that an electrocardiogram change of a patient having myocardial ischemia is mainly an ST-T change, but an ST-T change is not necessarily caused by myocardial ischemia. An ST-T change is related to a plurality of factors, and an ST-T change cannot be equivalent to myocardial ischemia or cardiac death. For example, influencing factors of an ST-T change may include a physiological factor, a pharmacological factor, an extracardiac disease, a heart disease, and the like. The physiological factor may include a body position, body temperature, excessive ventilation, anxiety, tachycardia, a neurogenic effect, physical exercise, age, or the like. The pharmacological factor may include digitalis, antiarrhythmia drugs, or antipsychotic drugs. The extracardiac disease may include an electrolyte disorder, a cerebrovascular accident, shock, anemia, an allergic reaction, infection, an endocrine disorder, an acute abdominal disorder, pulmonary embolism, or the like. The heart disease may include ischemic heart disease, primary cardiomyopathy, secondary myocardial change, pericardial disease, abnormal electrocardiogram, or the like.

The ST-T change may further include a primary ST-T change and a secondary ST-T change. The primary ST-T change refers to an ST-T change when ventricular depolarization does not change, and refers to an electrocardiogram ST-T change caused by abnormal ventricular repolarization that is caused by an abnormal myocardial condition. The primary ST-T change is clinically common in chronic coronary artery blood supply deficiency, angina, myocardial infarction, ventricular hypertrophy, myocarditis, pericarditis, cardiomyopathy, drug action, an electrolyte disorder, and the like. The secondary ST-T change refers to an ST-T change when ventricular depolarization changes, and refers to an electrocardiogram ST-T change caused by abnormal ventricular repolarization that is caused by abnormal ventricular depolarization. The secondary ST-T change is clinically common in ventricular hypertrophy, bundle branch block, ventricular preexcitation, ventricular excitement, paced heart rhythm, and the like.

It should be noted that this embodiment of this application may be applied to an electrocardiogram (electrocardiogram, ECG) signal collection device. For example, the ECG signal collection device may be a wearable device, for example, a smart watch, a smart chest strap, a single-lead ECG watch, a multi-lead ECG watch, a chest ECG sticker, or a single-lead or multi-lead ECG sticker. The ECG signal collection device may collect a single-lead or multi-lead ECG signal, and process the collected ECG signal, to obtain a coronary heart disease probability of a user. In addition, a most probable coronary heart disease lesion location may be further obtained. An ECG signal collection device having a display may further display, on the display, obtained related information such as a coronary heart disease probability of the user and a most probable coronary heart disease lesion location of the user. In addition, in some embodiments, one ECG signal collection device (referred to as a first device for short below) in at least two ECG signal collection devices may collect an ECG signal, and obtain an ECG signal collected by another ECG signal collection device. The first device performs processing on both the ECG signal collected by the first device and the obtained ECG signal collected by the another ECG signal collection device, to obtain related information such as a coronary heart disease probability of the user and a most probable coronary heart disease lesion location of the user.

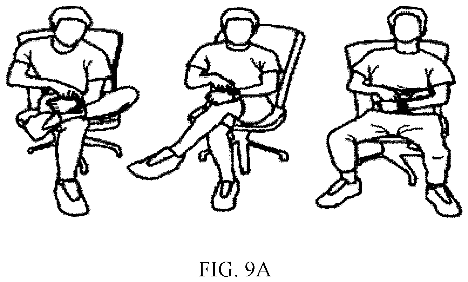

In some embodiments, an ECG signal collection device may alternatively collect only an ECG signal, and send the collected ECG signal to an ECG signal processing device for processing. The ECG signal processing device may process the obtained ECG signal, to obtain a coronary heart disease probability of the user. In addition, a most probable coronary heart disease lesion location may be further obtained. An ECG signal processing device having a display may further display, on the display, obtained related information such as a coronary heart disease probability of the user and a most probable coronary heart disease lesion location of the user. For example, the ECG signal processing device herein may be a mobile phone, a tablet, a computer, a television, or the like. It may be understood that, if the ECG signal processing device does not have a display, the related information such as the coronary heart disease probability of the user and the most probable coronary heart disease lesion location the user may be further sent to a display device for display. In an example, the ECG collection device is an ECG wearable watch. A user A wears the ECG wearable watch in a posture (an electrode in the watch is in contact with an ankle preset location, a leg preset location, or a chest preset location) shown in FIG. 9A, so that the ECG wearable watch can collect ECG signals of six leads on limbs. The ECG wearable watch may process the collected ECG signals of the six limb leads by using the method provided in this embodiment of this application, to obtain a coronary heart disease probability of the user. Alternatively, the ECG wearable watch may transmit the ECG signals of the six limb leads to the ECG signal processing device (for example, a mobile phone, an iPad, or a computer). The ECG signal processing device processes the collected ECG signals of the six limb leads by using the method provided in this embodiment of this application, to obtain a coronary heart disease probability of the user A.

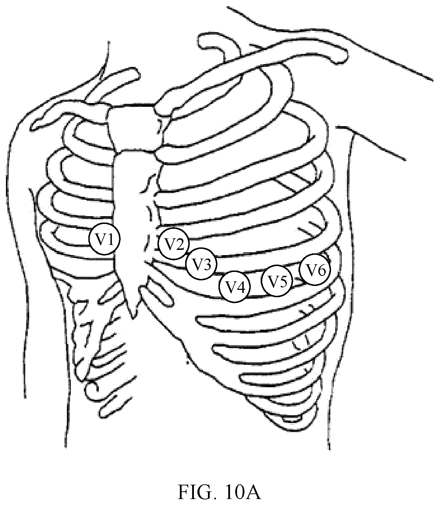

In an example, the ECG collection device is a heart rate chest strap, and the user wears the heart rate chest strap. As shown in FIG. 10A, the heart rate chest strap is in contact with preset locations (V1 to V6) on a user B, and may collect ECG signals of six leads on a chest. The heart rate chest strap may transmit the collected ECG signals of the six chest leads to the ECG signal processing device (for example, a mobile phone, an iPad, or a computer). The ECG signal processing device processes the collected ECG signals of the six chest leads by using the method provided in this embodiment of this application, to obtain a coronary heart disease probability of the user B.

In still another example, the ECG collection device is an ECG wearable watch and a heart rate chest strap, and a user C wears the ECG wearable watch and the heart rate chest strap. Based on the foregoing two examples, the ECG wearable watch may collect the ECG signals of the six limb leads, and the heart rate chest strap may collect the ECG signals of the six chest leads. The heart rate chest strap may transmit the collected ECG signals of the six chest leads to the ECG wearable watch. The ECG wearable watch may process the ECG signals of the twelve leads by using the method provided in this embodiment of this application, to obtain a coronary heart disease probability of the user C. Alternatively, the heart rate chest strap transmits the collected ECG signals of the six chest leads to the ECG signal processing device, and the ECG wearable watch transmits the ECG signals of the six limb leads to the ECG signal processing device. The ECG signal processing device processes the ECG signals of the twelve leads by using the method provided in this embodiment of this application, to obtain a coronary heart disease probability of the user C.

Embodiments of this application provide a method and an apparatus for determining a coronary heart disease probability, to estimate a coronary heart disease probability.

First, a first model M1, a second model M2, and a third model M3 need to be established.

The first model M1 is used to perform full-segment feature extraction on an original ECG signal. The second model M2 is used to perform reconstruction on a full-segment feature extracted by the first model M1, to obtain a reconstructed signal corresponding to the original ECG signal. The full-segment feature refers to a feature of an original ECG signal included within at least one complete periodicity. For example, features obtained from a start location of a first P wave to a start location of a second P wave in two adjacent heartbeats in an ECG are full-segment features within one periodicity. Alternatively, features obtained from a start location to an end location in an ECG graph obtained through one detection are full-segment features obtained through one detection.

For example, the first model M1 and the second model M2 may be established by using but not limited to the following methods. An ECG signal of each lead of a healthy population is selected as an original training signal, a full-segment feature of each original training signal is obtained, and the first model M1 is obtained through training based on the full-segment feature of each original training signal. For example, methods for obtaining the first model M1 through training may include but are not limited to a neural network, a random forest, and the like. The full-segment feature herein includes but is not limited to a one-dimensional feature, a two-dimensional feature, or a multi-dimensional feature. Further, signal reconstruction is performed based on the full-segment feature of each original training signal, and the second model M2 is obtained through training based on a reconstructed signal. For example, methods for obtaining the second model M2 through training include but are not limited to a neural network, a random forest, and the like. The healthy population may refer to a population having no coronary heart disease and another cardiovascular disease.

In some embodiments, an ECG signal of each lead of a healthy population is selected as an original training signal, and data preprocessing is performed on each original training signal. For example, the original training signal includes an ECG signal of a first lead. The ECG signal of the first lead may be represented by an N-dimensional signal [x1, x2, x3, ..., xN], where N is a positive integer. For example, xi in [x1, x2, x3, ..., xN] may be a signal amplitude at an i^{th} moment, or a value of a sampling point whose sampling order is i, where i is a positive integer. For example, if a sampling frequency is 100 Hz and sampling duration is 30s, N is equal to 3000. In other words, sampling is performed 100 times per second and the sampling duration is 30s, to obtain 3000 sampling points in total. Data preprocessing performed on the ECG signal of the first lead may include operations such as upsampling, downsampling, baseline removal, noise reduction, and filtering. A specific operation of data preprocessing is not limited in this embodiment of this application.

Then, the first model M1 is trained based on a preprocessed original training signal by using a machine learning method (including but not limited to a neural network, a random forest, and the like). For example, a full-segment feature of the ECG signal of the first lead is obtained based on a preprocessed ECG signal of the first lead. The full-segment feature of the ECG signal of the first lead may be represented by an M-dimensional signal [y1, y2, y3, ..., yM], where M is less than N, and M is a positive integer. The full-segment feature herein may include but is not limited to a one-dimensional feature, a two-dimensional feature, or a multi-dimensional feature. Parameters of the first model M1 are continuously improved through a training process of performing full-segment feature extraction on a large quantity of original training signals.

Further, signal reconstruction is performed based on a full-segment feature of each original training signal, and the second model M2 is trained by using a corresponding machine learning method (including but not limited to a neural network, a random forest, and the like). For example, an N-dimensional signal [z1, z2, z3, ..., zN] is reconstructed based on the full-segment feature [y1, y2, y3, ..., yM] of the ECG signal of the first lead. Parameters of the second model M2 are continuously improved through a training process of performing signal reconstruction on full-segment features of a large quantity of original training signals.

For example, the first model M1 and the second model M2 may not be distinguished, but are combined into one model, to obtain, from the ECG signals of the one or more leads, the reconstructed signal corresponding to the ECG signal of each lead.

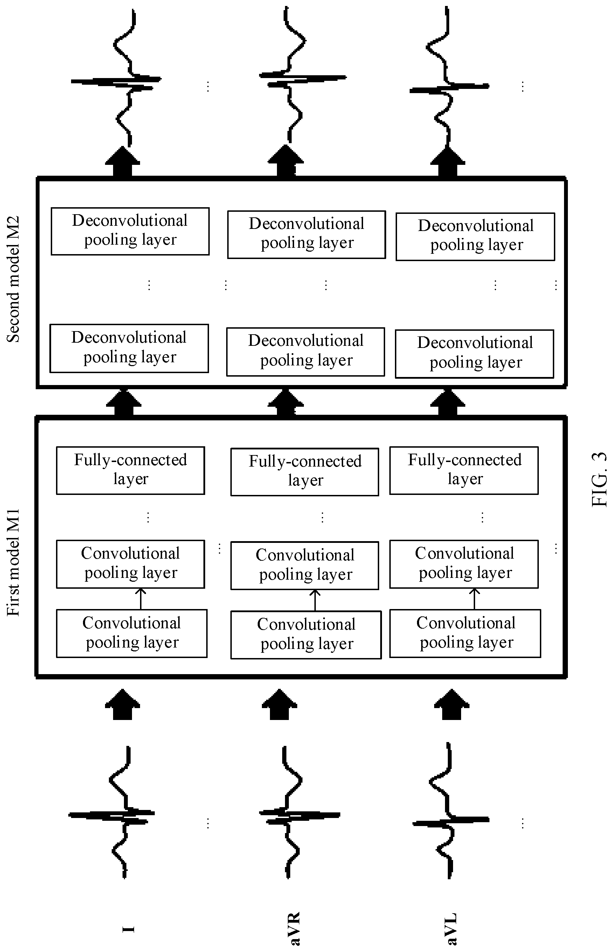

As shown in FIG. 3, signals on a leftmost side in FIG. 3 are ECG signals of leads of a healthy population. The ECG signals of the leads are processed by using the first model M1, where processing performed by using the first model M1 specifically includes processing at a plurality of convolutional pooling layers, a fully-connected layer, and the like, to extract a full-segment feature of the original signal. Further, signal reconstruction is performed by using the second model M2 based on the extracted full-segment feature, where the signal reconstruction specifically includes processing at a plurality of deconvolutional pooling layers, to obtain reconstructed signals. In other words, signals on a rightmost side in FIG. 3 are reconstructed signals of the ECG signals of the leads.

For example, the N-dimensional signal [x1, x2, x3, ..., xN] may be processed at a plurality of convolutional pooling layers, a fully-connected layer, and the like, to obtain the M-dimensional signal [y1, y2, y3, ..., yM]; and the M-dimensional signal [y1, y2, y3, ..., yM] is processed at a plurality of deconvolutional pooling layers, to reconstruct the N-dimensional signal [z1, z2, z3, ..., zN]. Values of a same sequence number in the N-dimensional signal [x1, x2, x3, ..., xN] and the N-dimensional signal [z1, z2, z3, ..., zN] are signal amplitudes at a same moment. For example, x1 and z1 respectively correspond to an amplitude of an original training signal at a moment 1 and an amplitude of a reconstructed signal corresponding to the original training signal at the moment 1.

It should be noted that the first model M1 and the second model M2 process an entire segment of ECG signal, instead of merely an ST-T segment signal in an ECG signal. For example, all ECG signals (an average of 30 periodicities per person) within 30s are used as input signals, instead of using a partial signal in an ECG signal as an input signal.

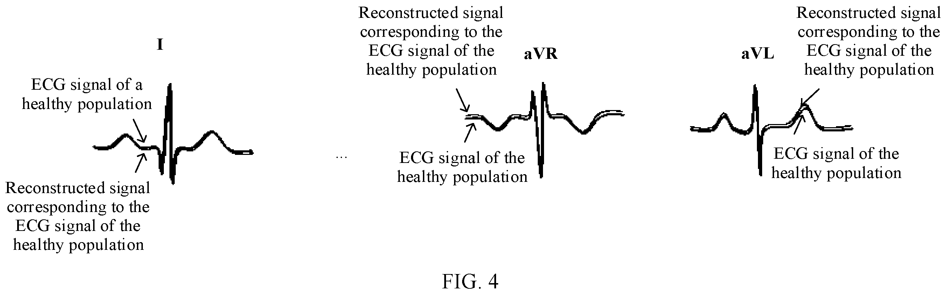

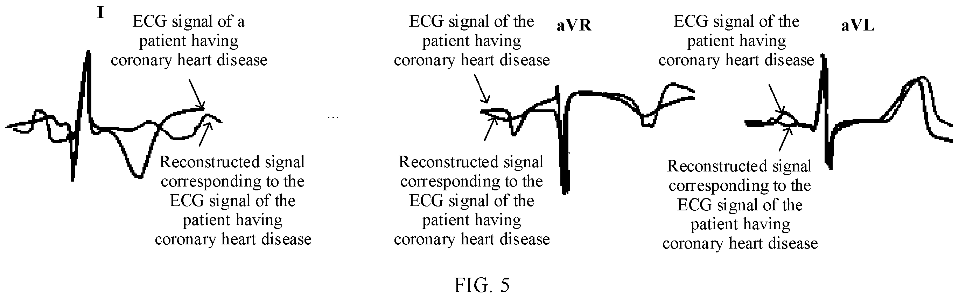

FIG. 4 is a schematic diagram of ECG signals of leads of a healthy population and reconstructed signals respectively corresponding to the ECG signals of the leads of the healthy population according to an embodiment of this application. FIG. 5 is a schematic diagram of ECG signals of leads of a patient having coronary heart disease and reconstructed signals respectively corresponding to the ECG signals of the leads of the patient having coronary heart disease according to an embodiment of this application.

It can be learned from FIG. 4 that, based on the first model M1 and the second model M2, the ECG signals of the leads of the healthy population are basically consistent with the reconstructed signals respectively corresponding to the ECG signals of the leads of the healthy population. As shown in FIG. 5, based on the first model M1 and the second model M2, there is a large difference between the ECG signals of the leads of the patient having coronary heart disease and reconstructed signals respectively corresponding to the ECG signals of the leads of the patient having coronary heart disease.

It may be understood that ECG signals of any two healthy users have a high similarity, but ECG signals of any two patients having coronary heart disease generally have a low similarity. In other words, ECG signals of patients having coronary heart disease are diversified, and it is difficult to obtain possible ECG signals of all patients having coronary heart disease. Specifically, different patients having coronary heart disease have different lesion locations and lesion degrees. Therefore, different patients having coronary heart disease have greatly different ECG signals. In addition, some patients having coronary heart disease may have another disease at the same time, and this may further affect ECG signals to some extent. For example, an ECG signal of a lead within one periodicity is divided into a plurality of segments. For a healthy user, each segment of ECG signal is within a corresponding interval range. However, for a patient having coronary heart disease, several segments of ECG signals are not within a corresponding interval range.

For example, full-segment features of an ECG signal of a healthy user are extracted according to the first model M1. ECG signals of different healthy users have a high similarity. Therefore, for different healthy users, extracted full-segment features meet a specific change regularity or a preset condition. Further, a reconstructed signal similar to the ECG signal of the healthy user may be obtained by performing signal reconstruction according to the second model M2 based on the extracted features. This is also a training purpose of the first model M1 and the second model M2. In other words, an ECG signal of a healthy user may be basically restored by using the first model M1 and the second model M2.

However, when full-segment features of an ECG signal of a patient having coronary heart disease are extracted according to the first model M1, these extracted full-segment features do not meet the foregoing change regularity or preset condition. In this case, a reconstructed signal obtained through performing signal reconstruction according to M2 based on the extracted features differ greatly from the original ECG signal of the patient having coronary heart disease. In other words, the original ECG signal of the patient having coronary heart disease cannot be restored.

The third model M3 may be established by using, but not limited to, the following methods. Methods for obtaining the third model M3 through training may include but are not limited to a neural network, a random forest, and the like.

Example 1: First, ECG signals of leads of a population having coronary heart disease, ECG signals of leads of a population having another type of disease, and ECG signals of leads of a healthy population are selected as original training signals. A reconstructed signal corresponding to each original training signal is obtained according to each original training signal, the first model M1, and the second model M2. The third model M3 is obtained through training based on the original training signal and the reconstructed signal corresponding to the original training signal. For example, the population having another type of disease may refer to a population having no coronary heart disease but having other cardiovascular diseases.

In some embodiments, first, ECG signals of leads of a population having coronary heart disease, ECG signals of leads of a population having another type of disease, and ECG signals of leads of a healthy population are selected as original training signals. A reconstructed signal corresponding to each original training signal is obtained according to each original training signal, the first model M1, and the second model M2. Then, a target signal corresponding to each original training signal is determined based on each original training signal and the corresponding reconstructed signal, where the target signal corresponding to each original training signal reflects a difference between the original training signal and the reconstructed signal corresponding to the original training signal.

Further, based on the target signal corresponding to each original training signal, the third model M3 is trained based on a label of each original training signal by using a machine learning method (including and not limited to a neural network, a random forest, and the like). A type label of the ECG signal of each lead of the population having coronary heart disease, a type label of the ECG signal of each lead of the population having another type of disease, and a type label of the ECG signal of each lead of a healthy population are different from each other. For example, the type label of the ECG signal of each lead of the population having coronary heart disease is coronary heart disease, the type label of the ECG signal of each lead of the population having another type of disease is another type of disease, and the type label of the ECG signal of each lead of the healthy population is health. Alternatively, the type label of the ECG signal of each lead of the population having coronary heart disease is a label 1, the type label of the ECG signal of each lead of the population having another type of disease is a label 2, and the type label of the ECG signal of each lead of the healthy population is a label 3. It may be understood that specific forms of the foregoing type labels are merely examples, and are not intended to limit this embodiment of this application.

For example, the original training signal includes an ECG signal of a first lead. The ECG signal of the first lead may be represented by an N-dimensional signal [x1, x2, x3, ..., xN], and a reconstructed signal corresponding to the ECG signal of the first lead may be represented by an N-dimensional signal [z1, z2, z3, ..., zN], where N is a positive integer. A difference operation between the ECG signal [x1, x2, x3, ..., xN] of the first lead and the corresponding reconstructed signal [z1, z2, z3, ..., zN] is performed, to obtain an N-dimensional signal [a1, a2, a3, ..., aN]. In this case, the N-dimensional signal [a1, a2, a3, ..., aN] is recorded as a target signal corresponding to the ECG signal of the first lead. Similarly, a corresponding target signal determined by a difference between another original training signal and a corresponding reconstructed signal may be obtained. Further, for the obtained target signal corresponding to each original training signal, the third model M3 is trained based on a label (for example, coronary heart disease, another type of disease, or health) of each original training signal by using a machine learning method (including and not limited to a neural network, a random forest, and the like). Parameters of the third model M3 are continuously improved through a process of training target signals corresponding to a large quantity of original training signals based on type labels of the original training signals.

Based on the third model M3 obtained in the manner in Example 1, an ECG signal of a user and a reconstructed signal corresponding to the ECG signal are used to determine a target signal corresponding to the ECG signal. The target signal corresponding to the ECG signal is input into the third model M3, so that a probability that the ECG signal is an ECG signal of a population having coronary heart disease, a probability that the ECG signal is an ECG signal of a population having another type of disease, and a probability that the ECG signal is an ECG signal of a healthy population can be obtained. Further, the probabilities may be respectively used as a coronary heart disease probability of the user, an another disease probability of the user, and a probability that the user belongs to a healthy group. A sum of the coronary heart disease probability of the user, the another disease probability of the user, and the probability that the user belongs to a healthy group is 1.

For example, a target signal is determined based on an ECG signal of an I lead of the user and a corresponding reconstructed signal. It is assumed that 1000 features may be extracted from the target signal by using the third model, and it may be determined that 700 features of the 1000 features match features of a target signal corresponding to an ECG signal of an I lead of a patient having coronary heart disease, 200 features match features of a target signal corresponding to an ECG signal of an I lead of a healthy user, and 100 features match features of a target signal corresponding to an ECG signal of an I lead of a patient having another disease. Therefore, a result output by the third model is as follows: A coronary heart disease probability of the user is 70%, a probability that the user belongs to a healthy population is 20%, and an another type of disease probability of the user is 10%.

It should be noted that, even if a target signal is determined based on an ECG signal of a patient having coronary heart disease in an original training signal and a corresponding reconstructed signal, and then the target signal is input into the third model, an obtained coronary heart disease probability of the user may not be 100%.

Example 2: First, ECG signals of leads of a population having coronary heart disease and ECG signals of leads of a healthy population are selected as original training signals. A reconstructed signal corresponding to each original training signal is obtained according to each original training signal, the first model M1, and the second model M2. The third model M3 is obtained through training based on the original signal and the reconstructed signal corresponding to the original signal.

In some embodiments, first, ECG signals of leads of a population having coronary heart disease and ECG signals of leads of a healthy population are selected as original training signals. A reconstructed signal corresponding to each original training signal is obtained according to each original training signal, the first model M1, and the second model M2. Then, a target signal corresponding to each original training signal is determined based on each original training signal and the corresponding reconstructed signal, where the target signal corresponding to each original training signal reflects a difference between the original training signal and the reconstructed signal corresponding to the original training signal.

Further, based on the target signal corresponding to each original training signal, the third model M3 is trained based on a label of each original training signal by using a machine learning method (including and not limited to a neural network, a random forest, and the like). A type label of the ECG signal of each lead of the population having coronary heart disease and a type label of the ECG signal of each lead of a healthy population are different from each other. For example, the type label of the ECG signal of each lead of the population having coronary heart disease is coronary heart disease, and the type label of the ECG signal of each lead of the healthy population is health. Alternatively, the type label of the ECG signal of each lead of the population having coronary heart disease is a label 1, and the type label of the ECG signal of each lead of the healthy population is a label 2. It may be understood that specific forms of the foregoing type labels are merely examples, and are not intended to limit this embodiment of this application.

For example, the original training signal includes an ECG signal of a first lead. The ECG signal of the first lead may be represented by an N-dimensional signal [x1, x2, x3, ..., xN], and a reconstructed signal corresponding to the ECG signal of the first lead may be represented by an N-dimensional signal [z1, z2, z3, ..., zN], where N is a positive integer. A difference operation between the ECG signal [x1, x2, x3, ..., xN] of the first lead and the corresponding reconstructed signal [z1, z2, z3, ..., zN] is performed, to obtain an N-dimensional signal [a1, a2, a3, ..., aN]. In this case, the N-dimensional signal [a1, a2, a3, ..., aN] is recorded as a target signal corresponding to the ECG signal of the first lead. Similarly, a corresponding target signal determined by a difference between another original training signal and a corresponding reconstructed signal may be obtained. Further, for the obtained target signal corresponding to each original training signal, the third model M3 is trained based on a type label (for example, coronary heart disease, another type of disease, or health) of each original training signal by using a machine learning method (including and not limited to a neural network, a random forest, and the like). Parameters of the third model M3 are continuously improved through a process of training target signals corresponding to a large quantity of original training signals based on type labels of the original training signals.

Based on the third model M3 obtained in the manner in Example 2, an ECG signal of a user and a reconstructed signal corresponding to the ECG signal are used to determine a target signal corresponding to the ECG signal. The target signal corresponding to the ECG signal is input into the third model M3, so that a probability that the ECG signal is an ECG signal of a population having coronary heart disease and a probability that the ECG signal is an ECG signal of a healthy population can be obtained. Further, the probabilities may be respectively used as a coronary heart disease probability of the user and a probability that the user belongs to a healthy group. A sum of the coronary heart disease probability of the user and the probability that the user belongs to a healthy group is 1.

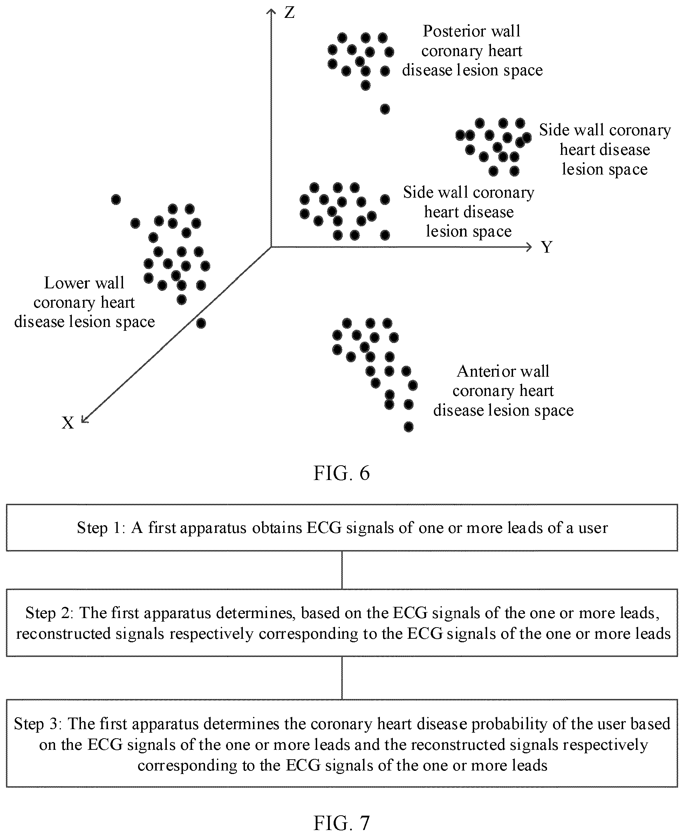

Based on the third model, if coronary heart disease corresponding to each patient having coronary heart disease has been determined through diagnosis, for example, coronary heart disease at a posterior wall, a side wall, a lower wall, anterior wall, or the like, or coronary heart disease at a left coronary main artery, an anterior descending branch, a circumflex branch, a right coronary artery, or the like, coronary heart disease lesion space may be obtained through training with reference to diagnostic labels (for example, a posterior wall, a side wall, a lower wall, or an anterior wall; or a left coronary main artery, an anterior descending branch, a circumflex branch, or a right coronary artery) of ECG signals of leads in the patient having coronary heart disease. Specifically, target signals respectively corresponding to the ECG signals of the leads of the patient having coronary heart disease are processed by using the third model, to extract a first feature (for example, a fully-connected layer feature). **In** addition, reduction of dimensions is performed on the first feature, where the dimensions include but are not limited to three dimensions. The coronary heart disease lesion space may be obtained through training with reference to the diagnostic labels of the ECG signals of the leads in the patient having coronary heart disease. FIG. 6 shows a schematic diagram of a 3-dimensional coronary heart disease lesion space. Target signals respectively corresponding to ECG signals of leads of a patient having coronary heart disease are determined by the ECG signals of the leads of the patient having coronary heart disease and reconstructed signals respectively corresponding to the ECG signals of the leads of the patient having coronary heart disease. An X-axis, a Y-axis, and a Z-axis in FIG. 6 may be understood as the following meanings but are not limited to the following meanings. The X-axis, the Y-axis, and the Z-axis may be used to identify a simulated three-dimensional space of a heart; the X-axis, the Y-axis, and the Z-axis may be used to identify a lesion degree of a limb lead, a lesion degree of a thoracic lead, and a management lesion degree of limb and thoracic leads; or the X-axis, the Y-axis, and the Z-axis may be used to identify an ST lesion, a T lesion, and a P-wave lesion. Therefore, according to the third model M3, the first feature (for example, a fully-connected layer feature) may be determined based on the ECG signals of the user and the reconstructed signals corresponding to the ECG signals. In addition, reduction of dimensions (for example, reducing to three dimensions) is performed on the first feature. A most probable lesion location of the user is determined based on location information of the first feature in coronary heart disease lesion space shown in FIG. 6. As shown in FIG. 7, the following uses a first apparatus as an example to describe a specific process of determining a coronary heart disease probability of a user. The first apparatus may be an ECG signal collection device or an ECG signal processing device, or a chip in an ECG signal collection device or an ECG signal processing device.

Step 1: The first apparatus obtains ECG signals of one or more leads of the user.

When the first apparatus is an ECG signal collection device, the first apparatus may collect an ECG signal, or the first apparatus may collect, at the same time, an ECG signal collected by another ECG signal collection device.

When the first apparatus is an ECG signal processing device, the first apparatus collects an ECG signal collected by another ECG signal collection device.

Step 2: The first apparatus determines, based on the ECG signals of the one or more leads, reconstructed signals respectively corresponding to the ECG signals of the one or more leads. The ECG signals of the one or more leads include an ECG signal of a first lead, and a reconstructed signal corresponding to the ECG signal of the first lead is obtained by performing signal reconstruction based on a feature of the ECG signal of the first lead.

The first apparatus extracts a feature, for example, a full-segment feature, of an ECG signal of each lead in the ECG signals of the one or more leads, and performs signal reconstruction based on the feature of the ECG signal of each lead, to obtain a reconstructed signal corresponding to the ECG signal of each lead.

For example, full-segment feature extraction is performed on the ECG signal of each lead in the ECG signals of the one or more leads based on the first model M1, and reconstruction is performed, based on the second model M2, on the full-segment feature extracted from the ECG signal of each lead in the ECG signals of the one or more leads, to obtain the reconstructed signal corresponding to the ECG signal of each lead.

In some embodiments, after reconstructed signals respectively corresponding to the ECG signals of the one or more leads are determined, the first apparatus may further determine an abnormal signal in the ECG signals of the one or more leads based on the ECG signals of the one or more leads and the reconstructed signals respectively corresponding to the ECG signals of the one or more leads.

For example, if an ECG signal of any one lead and a reconstructed signal corresponding to ECG signal of the lead meet one or more of the following abnormal signal conditions, it may be determined that the ECG signal of the lead is an abnormal signal.

Condition 1: In differences between two segments of signals, an absolute value of a difference is greater than a threshold T1.

For example, if a greatest value in absolute values of differences between an ECG signal of an I lead and a reconstructed signal of the ECG signal of the I lead is 0.3 mV, and a threshold T1 corresponding to the I lead is 0.2 mV, the ECG signal of the I lead is an abnormal signal. Condition 2: In differences between two segments of signals, a normalized value (an absolute value of a difference/an unsigned greatest value) of an absolute value of a difference is greater than a threshold T2, where the unsigned greatest value is a greatest value in absolute values of amplitudes of an original signal in the two segments of signals.

Condition 3: A sum of squares of differences between two segments of signals is greater than a threshold T3.

Condition 4: A sum of squares of normalized values (a difference/an unsigned greatest value) is greater than a threshold T4.

Condition 5: A standard deviation of differences between two segments of signals is greater than a threshold T5.

Condition 6: A standard deviation of normalized values (a difference/an unsigned greatest value) is greater than a threshold T6.

Condition 7: An X1 percentile of absolute values of differences between two segments of signals is greater than a threshold T7.

Condition 8: An X2 percentile of normalized values (an absolute value of a difference/an unsigned greatest value) is greater than a threshold T8.

It may be understood that the foregoing conditions are merely examples, and another method may be used to determine an abnormal signal.

The threshold T1 to the threshold T8, X1, and X2 may be determined based on a statistical result, for example, a 99% quantile, of differences between ECG signals of original leads of a patient having coronary heart disease and reconstructed signals corresponding to the ECG signals of the original leads.

For example, if a reconstructed signal corresponding to an ECG signal of a first lead and the ECG signal of the first lead meet one or more of the foregoing Condition 1 to Condition 8, it is determined that the ECG signal of the first lead is an abnormal signal, or referred to as a signal of a lesion lead.

For another example, as shown in FIG. 5, ECG signals respectively corresponding to an I lead, an aVR lead, and an aVL lead meet one or more of the foregoing Condition 1 to Condition 8, and are abnormal signals.

It may be understood that the first apparatus may determine, based on the obtained ECG signal of each lead, whether the obtained ECG signal is an abnormal signal. If it is determined that there is no abnormal signal in the obtained ECG signals of the one or more leads, it may be determined that the user belongs to a healthy population, or a coronary heart disease probability of the user is 0. For example, a display shows that the user belongs to a healthy population, or the coronary heart disease probability of the user is 0. In addition, the first apparatus may not determine whether the ECG signal of each lead is an abnormal signal, and directly perform step 3.

Step 3: The first apparatus determines the coronary heart disease probability of the user based on the ECG signals of the one or more leads and the reconstructed signals respectively corresponding to the ECG signals of the one or more leads.

For example, when the coronary heart disease probability is high, the first apparatus may further suggest that the user go to a hospital for consultation with coronary heart disease. When the coronary heart disease probability is low, the first apparatus may further remind the user of performing ECG measurement again, or reserve a next ECG measurement for the user, to monitor a health status of the user.

It should be noted that a representation form of the coronary heart disease probability herein may be, but is not limited to, one of or a combination of more of the following manners.
1. The coronary heart disease probability may be a specific value.
2. The coronary heart disease probability may be represented as being high, medium or low. When the coronary heart disease probability may be represented as being high, medium, or low, if the coronary heart disease probability indicates a low probability, it may indicate that the user has a low coronary heart disease risk, or may indicate that the user belongs to a healthy population. A specific indication depends on a classification rule. For example, when the coronary heart disease probability is more than 70%, the coronary heart disease probability indicates a high probability. When the coronary heart disease probability ranges from 50% to 70%, the coronary heart disease probability indicates a medium probability. When the coronary heart disease probability ranges from 30% to 50%, the coronary heart disease probability indicates a low probability. When the coronary heart disease probability is less than 30%, it is determined that the user belongs to a healthy population. Further, when the coronary heart disease probability indicates a high or medium probability, the first apparatus may further suggest that the user go to a hospital for examination. When the coronary heart disease probability indicates a low probability, the first apparatus may further remind the user of performing ECG measurement again, or reserve a next ECG measurement for the user, to monitor a health status of the user. For another example, when the coronary heart disease probability is more than 65%, the coronary heart disease probability indicates a high probability. When the coronary heart disease probability ranges from 35% to 65%, the coronary heart disease probability indicates a medium probability. When the coronary heart disease probability is less than 35%, the coronary heart disease probability indicates a low probability. In this case, that the coronary heart disease probability indicates a low probability represents that the user belongs to a healthy population.

It may be understood that the foregoing specific values are merely examples, and are not intended to limit this embodiment of this application.

In addition, the coronary heart disease probability may be represented by using more categories. This is not limited in this embodiment of this application. For example, the coronary heart disease probability is represented as A+, A, A-, B+, B, B-, or C in descending order.

3. The coronary heart disease probability may alternatively be represented by using a chart like a column chart or a sector chart.

In some embodiments, the first apparatus determines the coronary heart disease probability of the user by using the third model based on the ECG signals of the one or more leads and the reconstructed signals respectively corresponding to the ECG signals of the one or more leads. In this case, the first apparatus does not screen a signal input into the third model.

In some embodiment, the first apparatus determines, based on an abnormal signal and a reconstructed signal corresponding to the abnormal signal, the coronary heart disease probability of the user by using the third mode. In this case, the first apparatus first screens out the abnormal signal and the reconstructed signal corresponding to the abnormal signal, and then performs processing by using the third model.

It may be understood that, if the first apparatus does not screen signals input into the third model, a calculation amount of the signals input into the third model may be large, but time and workload consumed for screening the signals input into the third model in an early stage are reduced.

However, if the first apparatus first screens out the abnormal signal and the reconstructed signal corresponding to the abnormal signal, and then performs processing by using the third model, a calculation amount of the third model can be reduced. **In** addition, when no abnormal signal is found through screening, it may be directly learned that the user is a user having no coronary heart disease, and signal processing does not need to be performed by using the third model.

**In** an example, the first apparatus determines, based on the abnormal signal and the reconstructed signal corresponding to the abnormal signal, a target signal corresponding to the abnormal signal, where the target signal corresponding to the abnormal signal reflects a difference between the abnormal signal and the reconstructed signal corresponding to the abnormal signal. For example, it is assumed that the ECG signal of the first lead is an abnormal signal. The ECG signal of the first lead may be represented by an N-dimensional signal [x1, x2, x3, ..., xN], and a reconstructed signal corresponding to the ECG signal of the first lead may be represented by an N-dimensional signal [z1, z2, z3, ..., zN], where N is a positive integer. A difference operation between the ECG signal [x1, x2, x3, ..., xN] of the first lead and the corresponding reconstructed signal [z1, z2, z3, ..., zN] is performed, to obtain an N-dimensional signal [a1, a2, a3, ..., aN]. **In** this case, the N-dimensional signal [a1, a2, a3, ..., aN] is recorded as a target signal corresponding to the ECG signal of the first lead.

The first apparatus determines, based on the target signal corresponding to the abnormal signal and by using the third model, the coronary heart disease probability of the user, the another disease probability of the user, and the probability that the user belongs to a healthy population. For example, a sum of a coronary heart disease probability of the user, an another disease probability of the user, and a probability that the user belongs to a healthy population is 1.

In this case, the third model is obtained by using the solution in Example 1 in the foregoing paragraphs related to the third model. Repeated parts are not described again.

For example, the first apparatus determines, based on the abnormal signal and the reconstructed signal corresponding to the abnormal signal and by using the third model, that the coronary heart disease probability of the user is 70%, the another disease probability of the user is 15%, and the probability that the user belongs to a healthy population is 15%. In this case, the first apparatus may display only the coronary heart disease probability 70% of the user; or display the coronary heart disease probability 70% of the user, the another disease probability 15% of the user, and the probability 15% that the user belongs to a healthy population; or display the high coronary heart disease probability of the user; or display a column chart showing the coronary heart disease probability of the user; or display sector charts corresponding to the coronary heart disease probability of the user, the another disease probability of the user, the probability that the user belongs to a healthy population, or the like. This is not limited in this embodiment of this application.

For example, the first apparatus may further focus only on the coronary heart disease probability of the user, and does not remind that the another disease probability of the user is high. Alternatively, if the another disease probability of the user is high, the first apparatus may further remind the user of going to a hospital for further examination.

It may be understood that existence of an abnormal signal does not necessarily indicate that a user corresponding to the abnormal signal is a patient having coronary heart disease. The user may alternatively be a patient having another disease or the user belongs to a healthy population.

In an example, the first apparatus determines, based on the abnormal signal and the reconstructed signal corresponding to the abnormal signal, a target signal corresponding to the abnormal signal, where the target signal corresponding to the abnormal signal reflects a difference between the abnormal signal and the reconstructed signal corresponding to the abnormal signal. For example, it is assumed that the ECG signal of the first lead is an abnormal signal. The ECG signal of the first lead may be represented by an N-dimensional signal [x1, x2, x3, ..., xN], and a reconstructed signal corresponding to the ECG signal of the first lead may be represented by an N-dimensional signal [z1, z2, z3, ..., zN], where N is a positive integer. A difference operation between the ECG signal [x1, x2, x3, ..., xN] of the first lead and the corresponding reconstructed signal [z1, z2, z3, ..., zN] is performed, to obtain an N-dimensional signal [a1, a2, a3, ..., aN]. In this case, the N-dimensional signal [a1, a2, a3, ..., aN] is recorded as a target signal corresponding to the ECG signal of the first lead.

The first apparatus determines, based on the target signal corresponding to the abnormal signal and by using the third model, the coronary heart disease probability of the user and the probability that the user belongs to a healthy population. For example, a sum of a coronary heart disease probability of the user and a probability that the user belongs to a healthy group is 1.

In this case, the third model is obtained by using the solution in Example 2 in the foregoing paragraphs related to the third model. Repeated parts are not described again.

In addition, in some embodiments, the first apparatus determines, based on the abnormal signal and the reconstructed signal corresponding to the abnormal signal, a first feature by using the third model; determines location information of the first feature in coronary heart disease lesion space based on the first feature; and determines a most probable coronary heart disease lesion location of the user based on the location information of the first feature in the coronary heart disease lesion space. For example, the first feature may be a fully-connected layer feature. For a manner of determining the coronary heart disease lesion space, refer to the foregoing related paragraphs. Repeated parts are not described again.

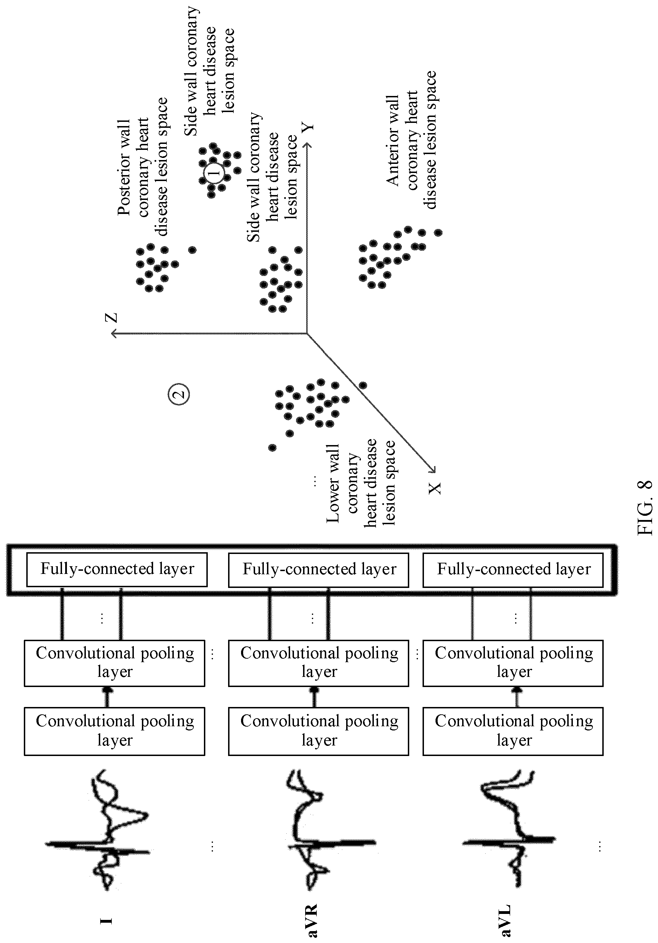

As shown in FIG. 8, the first apparatus inputs an abnormal signal and a reconstructed signal corresponding to the abnormal signal into the third model M3, to be processed, for example, at a convolutional pooling layer and a fully-connected layer, so that location information of fully-connected layer features in coronary heart disease lesion space may be determined. A most probable coronary heart disease lesion location may be determined based on the location information of the fully-connected layer features in the coronary heart disease lesion space.

For example, the first apparatus inputs the abnormal signal and the reconstructed signal corresponding to the abnormal signal into the third model M3, to determine a feature 1 in FIG. 8. Based on coordinates of the feature 1, it may be determined that the feature 1 falls into posterior wall lesion space. Further, it may be learned that a most probable coronary heart disease lesion location of the user is a posterior wall. In other words, the user may be a patient having posterior wall lesion coronary heart disease. For another example, the first apparatus inputs the abnormal signal and the reconstructed signal corresponding to the abnormal signal into the third model M3, to determine a feature 2 in FIG. 8. If it is determined that the feature 2 does not fall into any coronary heart disease lesion space, it may be learned that the user may be a patient having another disease but not a patient having coronary heart disease.

Similarly, the third model is also applicable to a case in which the first apparatus directly determines the first feature based on the ECG signals of the one or more leads and the reconstructed signals corresponding to the ECG signals of the one or more leads; determines location information of the first feature in coronary heart disease lesion space based on the first feature; and determines a most probable coronary heart disease lesion location of the user based on the location information of the first feature in the coronary heart disease lesion space. Repeated parts are not described again.

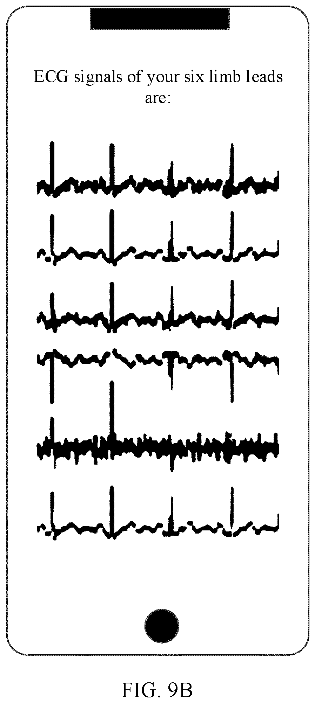

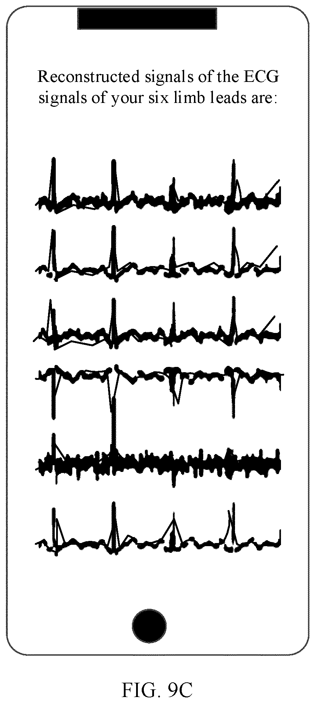

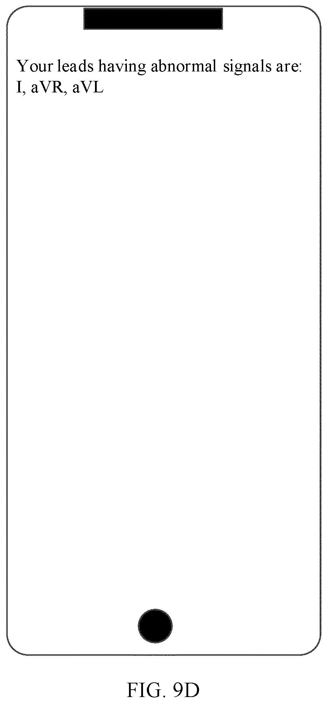

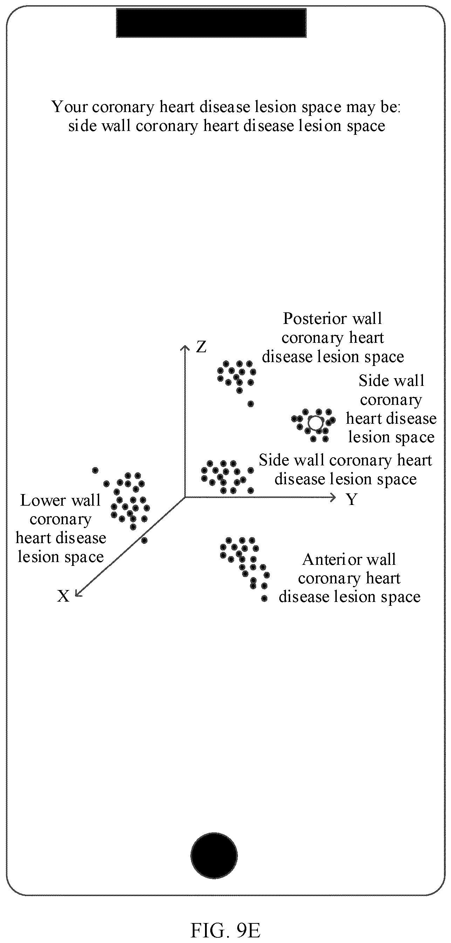

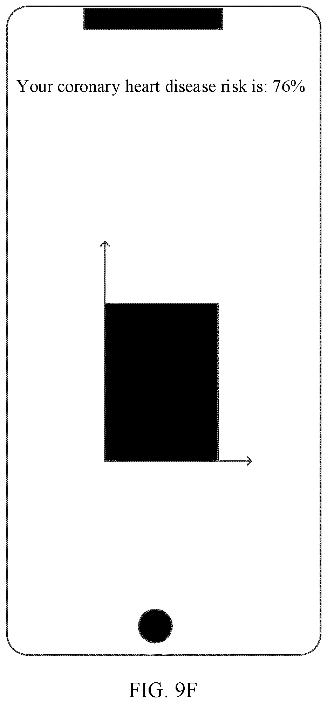

The following describes the embodiment shown in FIG. 7 with reference to a specific embodiment. Example 1: The user A collects an ECG signal of one lead or ECG signals of some leads in ECG signals of limb leads by using an ECG wearable watch. For example, the user A wears the ECG wearable watch in a posture (an electrode in the watch is in contact with an ankle preset location, a leg preset location, or a chest preset location) shown in FIG. 9A, so that the ECG wearable watch can collect ECG signals of six limb leads. For details, refer to ECG signals of six limb leads shown in FIG. 9B. The ECG wearable watch separately performs full-segment feature extraction on the obtained ECG signals of the six leads by using the first model M1, and performs reconstruction, by using the second model M2, on full-segment features extracted from the ECG signals of the leads, to obtain reconstructed signals corresponding to the ECG signals of the leads, as shown in FIG. 9C. The ECG wearable watch determines at least one abnormal signal based on the obtained ECG signals of the leads and the reconstructed signals corresponding to the ECG signals of the leads. For example, ECG signals respectively corresponding to an aVR lead and an aVL lead are abnormal signals, as shown in FIG. 9D. Further, the ECG wearable watch determines, by using the third model M3 and based on the abnormal signal and the reconstructed signal corresponding to the abnormal signal, that a coronary heart disease probability of the user A is 76% (as shown in FIG. 9F). In addition, fully-connected layer features and location information of the fully-connected layer features in coronary heart disease lesion space may be further determined, to further obtain a most probable coronary heart disease lesion location of the user A. For example, the fully-connected layer features fall into side wall coronary heart disease lesion space, as shown in FIG. 9E. Further, the ECG wearable device may further suggest the user go to a hospital for consultation on coronary heart disease.

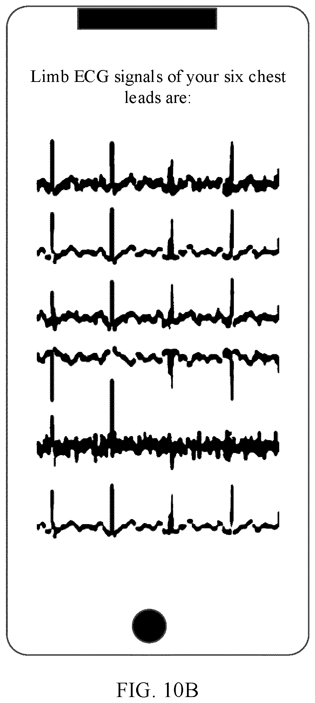

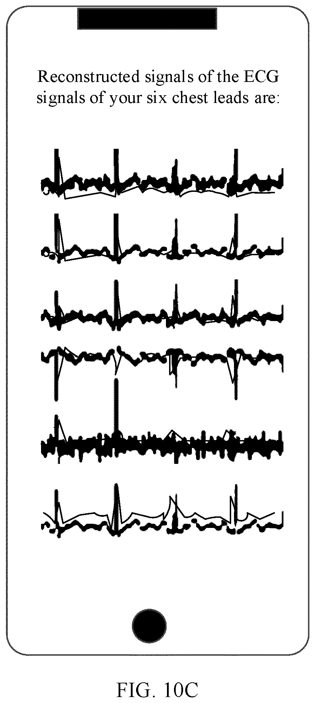

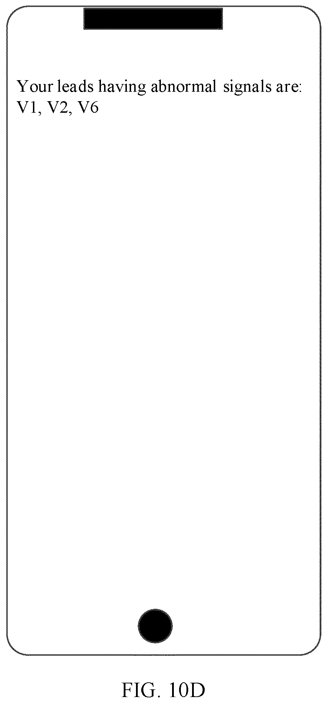

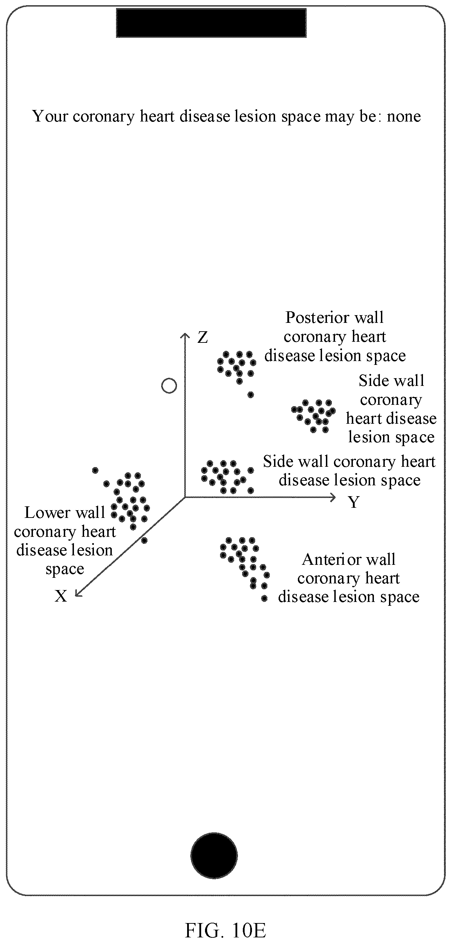

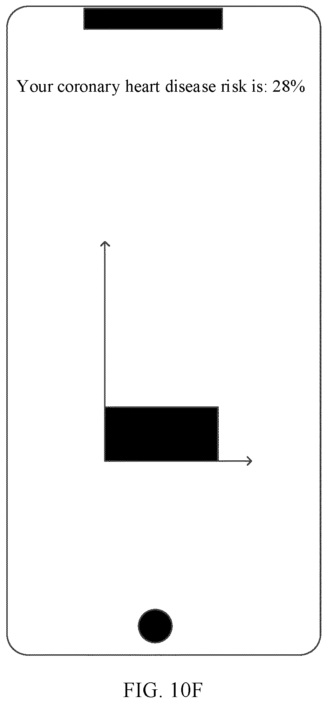

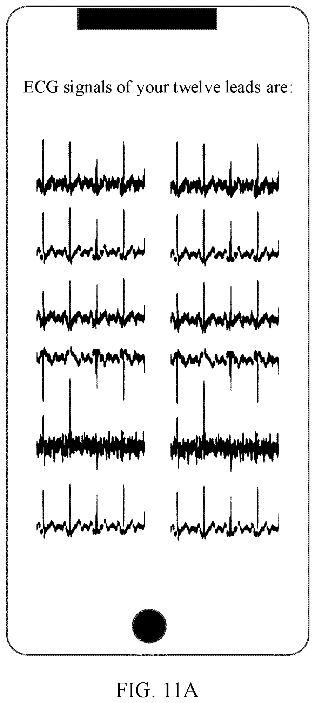

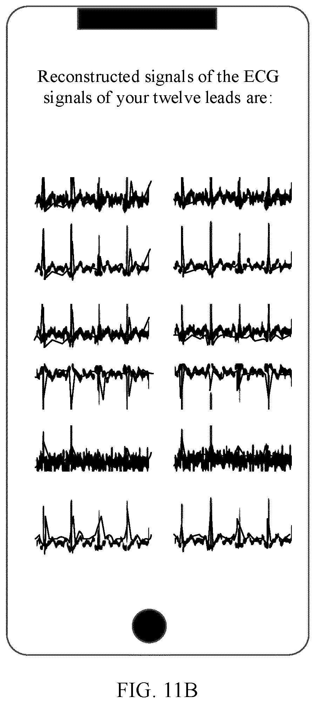

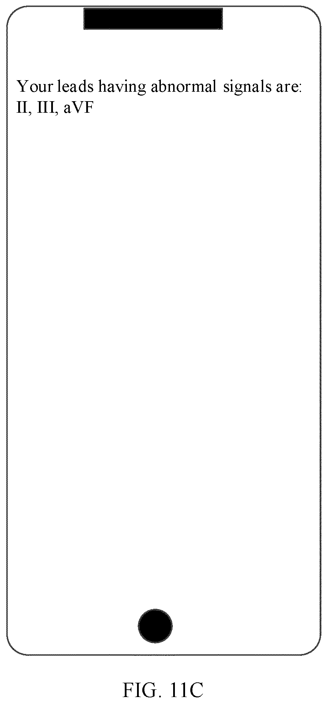

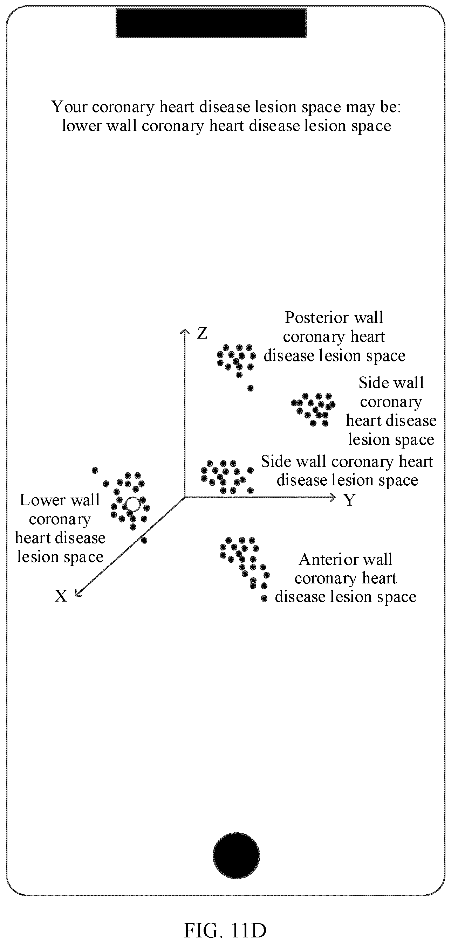

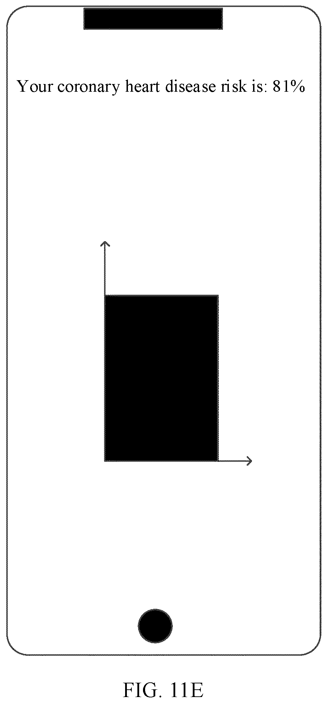

Example 2: The user B collects an ECG signal of one lead or ECG signals of some leads in ECG signals of chest leads by using a heart rate chest strap. For example, the user wears the heart rate chest strap. As shown in FIG. 10A, the heart rate chest strap is in contact with preset locations (V1 to V6) on the user B, and may collect ECG signals of six chest leads. FIG. 10B is a schematic diagram of the ECG signals of the six chest leads. The heart rate chest strap may transmit the collected ECG signals of the six chest leads to an ECG signal processing device, for example, a mobile phone of the user B. The mobile phone of the user B separately performs full-segment feature extraction on the obtained ECG signals of the six leads by using the first model M1, and performs reconstruction, by using the second model M2, on full-segment features extracted from the ECG signals of the leads, to obtain reconstructed signals corresponding to the ECG signals of the leads, as shown in FIG. 10C. The ECG wearable device determines at least one abnormal signal based on the obtained ECG signals of the leads and the reconstructed signals corresponding to the ECG signals of the leads. For example, ECG signals respectively corresponding to a V1 lead, a V2 lead, and a V6 lead are abnormal signals, as shown in FIG. 10D. Further, the mobile phone of the user B inputs an abnormal signal and a reconstructed signal corresponding to the abnormal signal into the third model M3, and determines that the ECG wearable device inputs the abnormal signal and the reconstructed signal corresponding to the abnormal signal into the third model M3, to determine that a coronary heart disease probability of the user B is 28%, an another disease probability is 52%, as shown in FIG. 10F. In addition, fully-connected layer features and location information respectively corresponding to the fully-connected layer features in coronary heart disease lesion space may be further determined. For example, the fully-connected layer features do not fall into any coronary heart disease lesion space, as shown in FIG. 10E. Further, the mobile phone of the user B may further remind the user B that although a coronary heart disease risk is low, there is an another disease risk, and suggest the user go to a hospital for further examination. Example 3: The user C collects, by using the ECG wearable watch, an ECG signal of one lead or ECG signals of some leads in the ECG signals of the limb leads, and collects, by using the heart rate chest strap, an ECG signal of one lead or ECG signals of some leads in the ECG signals of the chest leads. For example, the user C wears the ECG wearable watch and the heart rate chest strap. Based on the foregoing two examples, the ECG wearable watch may collect the ECG signals of the six limb leads, and the heart rate chest strap may collect the ECG signals of the six chest leads. The heart rate chest strap sends the ECG signals of the six chest leads to the ECG wearable watch. Therefore, the ECG wearable watch obtains ECG signals of twelve leads, and the ECG signals include the ECG signals of the six limb leads and the ECG signals of the six chest leads. FIG. 11A is a schematic diagram of the ECG signals of the twelve leads. The ECG wearable watch separately performs full-segment feature extraction on the obtained ECG signals of the twelve leads by using the first model M1, and performs reconstruction, by using the second model M2, on full-segment features extracted from the ECG signals, to obtain reconstructed signals corresponding to the ECG signals, as shown in FIG. 11B. The ECG wearable watch determines at least one abnormal signal based on the obtained ECG signals and the reconstructed signals corresponding to the ECG signals. For example, ECG signals respectively corresponding to an II lead, an III lead, and an aVF lead are abnormal signals, as shown in FIG. 11C. Further, the ECG wearable watch inputs an abnormal signal and the abnormal signal into the third model M3, to determine that a coronary heart disease probability of the user C is 81%, as shown in FIG. 11E. In addition, fully-connected layer features and location information respectively corresponding to the fully-connected layer features in coronary heart disease lesion space may be further determined, to obtain a most probable coronary heart disease lesion location of the user C. For example, the fully-connected layer features fall into low wall coronary heart disease lesion space, as shown in FIG. 11D. Further, the ECG wearable device may further suggest the user go to a hospital for consultation on coronary heart disease.

It may be understood that, to implement functions in the foregoing embodiments, a person skilled in the art should be easily aware that, with reference to the units and method steps in the examples described in embodiments disclosed in this application, this application can be implemented by hardware or a combination of hardware and computer software. Whether a function is performed by using hardware or hardware driven by computer software depends on a particular application scenario and design constraint of the technical solutions.

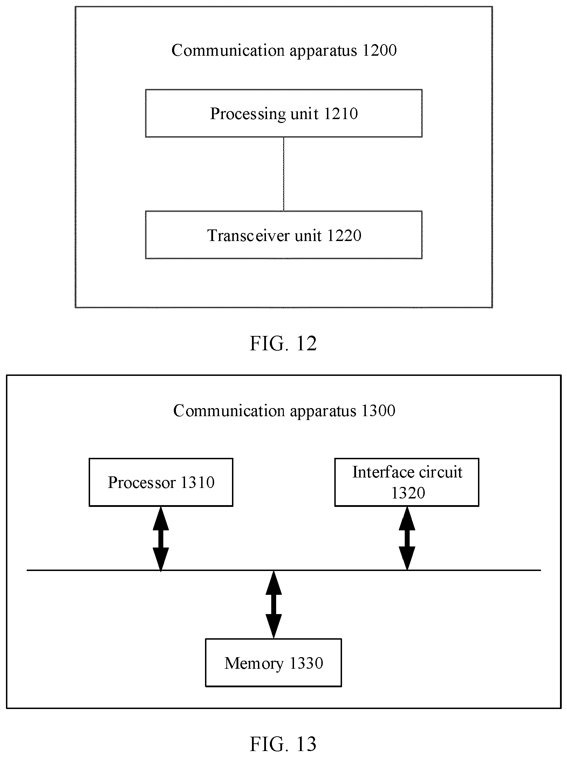

FIG. 12 and FIG. 13 are schematic diagrams of structures of possible communication apparatuses. These communication apparatuses may be configured to implement functions of the first apparatus in the foregoing method embodiments, and therefore can also implement beneficial effects of the foregoing method embodiments. In this embodiment of this application, the communication apparatus may be an ECG signal collection device or an ECG signal processing device, or may be a module (for example, a chip) applied to an ECG signal collection device or an ECG signal processing device.

As shown in FIG. 12, a communication apparatus 1200 includes a processing unit 1210 and a transceiver unit 1220. The communication apparatus 1200 is configured to implement a function of the first apparatus in the method embodiment shown in FIG. 7.

When the communication apparatus 1200 is configured to implement the function of the first apparatus in the method embodiment shown in FIG. 12, the transceiver unit 1220 is configured to obtain ECG signals of one or more leads of a user, and the processing unit 1210 is configured to determine, based on the ECG signals of the one or more leads, reconstructed signals respectively corresponding to the ECG signals of the one or more leads. The ECG signals of the one or more leads include an ECG signal of a first lead, and a reconstructed signal corresponding to the ECG signal of the first lead is obtained by performing signal reconstruction based on a feature of the ECG signal of the first lead. A coronary heart disease probability of the user is determined based on the ECG signals of the one or more leads and the reconstructed signals respectively corresponding to the ECG signals of the one or more leads.

For example, the first apparatus may be an ECG signal collection device, and may be a wearable device, for example, a smart watch, a smart chest strap, a single-lead ECG watch, a multi-lead ECG watch, a chest ECG sticker, or a single-lead or multi-lead ECG sticker. The ECG signal collection device may collect a single-lead or multi-lead ECG signal, and process the collected ECG signal, to obtain related information such as a coronary heart disease probability of a user. A first apparatus having a display may further display, on the display, obtained related information such as a coronary heart disease probability of the user.

In addition, in some embodiments, the first apparatus may collect an ECG signal, and obtain an ECG signal collected by another ECG signal collection device. The first apparatus performs processing on both the ECG signal collected by the first device and the obtained ECG signal collected by the another ECG signal collection device, to obtain related information such as a coronary heart disease probability of the user.

The first apparatus may be an ECG signal processing device. The first apparatus may process the obtained ECG signal, to obtain related information such as a coronary heart disease probability of the user. A first apparatus having a display may further display, on the display, obtained related information such as a coronary heart disease probability of the user. For example, the first apparatus herein may be a tablet, a computer, a television, or the like. It may be understood that, if the first apparatus does not have a display, the related information such as the coronary heart disease probability of the user may be further sent to a display device for display.

In a possible design, the processing unit is configured to: when the coronary heart disease probability of the user is determined based on the ECG signals of the one or more leads and the reconstructed signals respectively corresponding to the ECG signals of the one or more leads, determine an abnormal signal in the ECG signals of the one or more leads based on the ECG signals of the one or more leads and the reconstructed signals respectively corresponding to the ECG signals of the one or more leads; and determine the coronary heart disease probability of the user based on the abnormal signal and a corresponding reconstructed signal.

In a possible design, the processing unit is configured to: when the reconstructed signals respectively corresponding to the ECG signals of the one or more leads are determined based on the ECG signals of the one or more leads, extract a feature of an ECG signal of each lead in the ECG signals of the one or more leads by using a first model; and perform signal reconstruction based on the feature of the ECG signal of each lead by using a second model, to obtain a reconstructed signal corresponding to the ECG signal of each lead.

The first model and the second model are obtained through training based on an ECG signal of each lead of a healthy population.

In a possible design, the processing unit is configured to: when the abnormal signal in the ECG signals of the one or more leads is determined based on the ECG signals of the one or more leads and the reconstructed signals respectively corresponding to the ECG signals of the one or more leads, determine that the ECG signal of the first lead is an abnormal signal if the ECG signal of the first lead and the corresponding reconstructed signal meet one or more of the following abnormal signal conditions: In differences between the ECG signal of the first lead and the corresponding reconstructed signal, an absolute value of a difference is greater than a first threshold. Alternatively, in differences between the ECG signal of the first lead and the corresponding reconstructed signal, a ratio of an absolute value of a difference to an unsigned greatest value is greater than a second threshold, where the unsigned greatest value is a greatest value in absolute values of amplitudes of the ECG signal of the first lead. Alternatively, a sum of squares of absolute values of differences between the ECG signal of the first lead and the corresponding reconstructed signal is greater than a third threshold. Alternatively, a sum of squares of ratios of absolute values of differences between the ECG signal of the first lead and the corresponding reconstructed signal to an unsigned greatest value is greater than a fourth threshold, where the unsigned greatest value is a greatest value in absolute values of amplitudes of the ECG signal of the first lead. Alternatively, a standard deviation of differences between the ECG signal of the first lead and the corresponding reconstructed signal is greater than a fifth threshold. Alternatively, a standard deviation of ratios of differences between the ECG signal of the first lead and the corresponding reconstructed signal to an unsigned greatest value is greater than a sixth threshold, where the unsigned greatest value is a greatest value in absolute values of amplitudes of the ECG signal of the first lead. Alternatively, an X1 percentile of absolute values of differences between the ECG signal of the first lead and the corresponding reconstructed signal is greater than a seventh threshold, where X1 is a preset value. Alternatively, an X2 percentile of ratios of absolute values of differences between the ECG signal of the first lead and the corresponding reconstructed signal to an unsigned greatest value is greater than an eighth threshold. X2 is a preset value, and the unsigned greatest value is a greatest value in absolute values of amplitudes of the ECG signal of the first lead.

In a possible design, the processing unit is configured to: when the coronary heart disease probability of the user is determined based on the abnormal signal and the corresponding reconstructed signal, determine a target signal based on the abnormal signal and the corresponding reconstructed signal, where the target signal reflects a difference between the abnormal signal and the corresponding reconstructed signal; and determine the coronary heart disease probability of the user based on the target signal by using a third model.

The third model is obtained through training based on a first signal set and a second signal set. The first signal set includes target signals respectively corresponding to ECG signals of leads of a patient having coronary heart disease, and the target signals respectively corresponding to the ECG signals of the leads of the patient having coronary heart disease are determined by the ECG signals of the leads of the patient having coronary heart disease and reconstructed signals respectively corresponding to the ECG signals of the leads of the patient having coronary heart disease. The second signal set includes target signals respectively corresponding to ECG signals of the leads of a healthy population, and the target signals respectively corresponding to the ECG signals of the leads of the healthy population are determined by the ECG signals of the leads of the healthy population and reconstructed signals respectively corresponding to the ECG signals of the leads of the healthy population.

In a possible design, the processing unit is configured to: when the coronary heart disease probability of the user is determined based on the abnormal signal and the corresponding reconstructed signal, determine a target signal based on the abnormal signal and the corresponding reconstructed signal, where the target signal reflects a difference between the abnormal signal and the corresponding reconstructed signal; and determine the coronary heart disease probability of the user based on the target signal by using a third model.

The third model is obtained through training based on a first signal set, a second signal set, and a third signal set. The first signal set includes target signals respectively corresponding to ECG signals of leads of a patient having coronary heart disease, and the target signals respectively corresponding to the ECG signals of the leads of the patient having coronary heart disease are determined by the ECG signals of the leads of the patient having coronary heart disease and reconstructed signals respectively corresponding to the ECG signals of the leads of the patient having coronary heart disease. The second signal set includes target signals respectively corresponding to ECG signals of leads of a healthy population, and the target signals respectively corresponding to the ECG signals of the leads of the healthy population are determined by the ECG signals of the leads of the healthy population and reconstructed signals respectively corresponding to the ECG signals of the leads of the healthy population. The third signal set includes target signals respectively corresponding to ECG signals of leads of a population having another disease, and the target signals respectively corresponding to the ECG signals of the leads of the population having another disease are determined by the ECG signals of the leads of the population having another disease and reconstructed signals respectively corresponding to the ECG signals of the leads of the population having another disease.

In a possible design, the processing unit is configured to: determine a first feature based on the abnormal signal and the corresponding reconstructed signal by using the third model; determine location information of the first feature in coronary heart disease lesion space based on the first feature; and determine a most probable coronary heart disease lesion location of the user based on the location information of the first feature in the coronary heart disease lesion space.

In a possible design, the coronary heart disease lesion space is obtained through training based on the first signal set and diagnostic labels of the ECG signals of the leads of the patient having coronary heart disease.

In a possible design, the first feature is a fully-connected layer feature.

For more detailed descriptions of the processing unit 1210 and the transceiver unit 1220, directly refer to related descriptions of the method embodiment shown in FIG. 7. Details are not described herein again.

As shown in FIG. 13, a communication apparatus 1300 includes a processor 1310 and an interface circuit 1320. The processor 1310 and the interface circuit 1320 are coupled to each other. It may be understood that the interface circuit 1320 may be a transceiver or an input/output interface. Optionally, the communication apparatus 1300 may further include a memory 1330, configured to store instructions executed by the processor 1310, input data required for running instructions by the processor 1310, or data generated after the processor 1310 runs instructions.

When the communication apparatus 1300 is configured to implement the method shown in FIG. 12, the processor 1310 is configured to implement a function of the processing unit 1210, and the interface circuit 1320 is configured to implement a function of the transceiver unit 1220.

It may be understood that the processor in embodiments of this application may be a central processing unit (Central Processing Unit, CPU), or may be another general-purpose processor, a digital signal processor (Digital Signal Processor, DSP), an application-specific integrated circuit (Application-Specific Integrated Circuit, ASIC), a field programmable gate array (Field Programmable Gate Array, FPGA), another programmable logic device, a transistor logic device, a hardware component, or any combination thereof. The general purpose processor may be a microprocessor or may be any regular processor.

The method steps in embodiments of this application may be implemented in a hardware manner, or may be implemented in a manner of executing software instructions by the processor. The software instructions may include a corresponding software module. The software module may be stored in a random access memory (Random Access Memory, RAM), a flash memory, a read-only memory (Read-Only Memory, ROM), a programmable read-only memory (Programmable ROM, PROM), an erasable programmable read-only memory (Erasable PROM, EPROM), an electrically erasable programmable read-only memory (Electrically EPROM, EEPROM), a register, a hard disk, a removable hard disk, a CD-ROM, or any other form of storage medium well-known in the art. For example, a storage medium is coupled to a processor, so that the processor can read information from the storage medium and write information into the storage medium. Certainly, the storage medium may be a component of the processor. The processor and the storage medium may be disposed in an ASIC. In addition, the ASIC may be located in a network device or a terminal device. Certainly, the processor and the storage medium may exist in the network device or the terminal device as discrete components.

All or some of the foregoing embodiments may be implemented by using software, hardware, firmware, or any combination thereof. When software is used to implement the embodiments, all or some of the embodiments may be implemented in a form of a computer program product. The computer program product includes one or more computer programs and instructions. When the computer programs or instructions are loaded and executed on a computer, all or some of the procedures or functions in embodiments of this application are executed. The computer may be a general-purpose computer, a dedicated computer, a computer network, a network device, user equipment, or another programmable apparatus. The computer programs or instructions may be stored in a computer-readable storage medium, or may be transmitted from a computer-readable storage medium to another computer-readable storage medium. For example, the computer programs or instructions may be transmitted from a website, computer, server, or data center to another website, computer, server, or data center in a wired manner or in a wireless manner. The computer-readable storage medium may be any usable medium that can be accessed by a computer, or a data storage device, like a server or a data center, integrating one or more usable media. The usable medium may be a magnetic medium, for example, a floppy disk, a hard disk, or a magnetic tape, may be an optical medium, for example, a digital video disc (digital video disc, DVD), or may be a semiconductor medium, for example, a solid state drive (solid state drive, SSD).

In various embodiments of this application, unless otherwise stated or there is a logic conflict, terms and/or descriptions in different embodiments are consistent and may be mutually referenced, and technical features in different embodiments may be combined based on an internal logical relationship thereof, to form a new embodiment.

In this application, "at least one" means one or more, and "a plurality of" means two or more. The term "and/or" describes an association relationship between associated objects and indicates that three relationships may exist. For example, A and/or B may indicate the following three cases: Only A exists, both A and B exist, and only B exists. A and B each may be singular or plural. In the text descriptions of this application, the character "/" generally indicates an "or" relationship between the associated objects. In a formula in this application, the character "/" indicates a "division" relationship between the associated objects.

It may be understood that various numbers in embodiments of this application are merely used for differentiation for ease of description, and are not used to limit the scope of embodiments of this application. The sequence numbers of the foregoing processes do not mean execution sequences, and the execution sequences of the processes should be determined based on functions and internal logic of the processes.

## Claims

1. A computer-implemented method for determining a coronary heart disease probability, wherein the method comprises:
obtaining (Step 1) pre-obtained electrocardiogram ECG signals of one or more leads of a user;
determining (Step 2), based on the ECG signals of the one or more leads, reconstructed signals respectively corresponding to the ECG signals of the one or more leads, wherein the ECG signals of the one or more leads comprise an ECG signal of a first lead, and a reconstructed signal corresponding to the ECG signal of the first lead is obtained by performing signal reconstruction based on a feature of the ECG signal of the first lead; and
determining (Step 3) a coronary heart disease probability of the user based on the ECG signals of the one or more leads and the reconstructed signals respectively corresponding to the ECG signals of the one or more leads, wherein the determining (Step 2), based on the ECG signals of the one or more leads, reconstructed signals respectively corresponding to the ECG signals of the one or more leads comprises:
extracting a feature of an ECG signal of each lead in the ECG signals of the one or more leads by using a first model; and
performing signal reconstruction by using a second model based on the feature of the ECG signal of each lead, to obtain a corresponding reconstructed signal, wherein
the first model and the second model are obtained through training based on an ECG signal of each lead of a healthy population, wherein the determining (Step 3) a coronary heart disease probability of the user based on the ECG signals of the one or more leads and the reconstructed signals respectively corresponding to the ECG signals of the one or more leads comprises:
determining an abnormal signal in the ECG signals of the one or more leads based on the ECG signals of the one or more leads and the corresponding reconstructed signals; and
determining the coronary heart disease probability of the user based on the abnormal signal and a corresponding reconstructed signal.

2. The method according to claim 1, wherein the determining an abnormal signal in the ECG signals of the one or more leads based on the ECG signals of the one or more leads and the reconstructed signals respectively corresponding to the ECG signals of the one or more leads comprises:
determining that the ECG signal of the first lead is an abnormal signal if the ECG signal of the first lead and the corresponding reconstructed signal meet one or more of the following abnormal signal conditions:
in differences between the ECG signal of the first lead and the corresponding reconstructed signal, an absolute value of a difference is greater than a first threshold; or
in differences between the ECG signal of the first lead and the corresponding reconstructed signal, a ratio of an absolute value of a difference to an unsigned greatest value is greater than a second threshold, wherein the unsigned greatest value is a greatest value in absolute values of amplitudes of the ECG signal of the first lead; or
a sum of squares of absolute values of differences between the ECG signal of the first lead and the corresponding reconstructed signal is greater than a third threshold; or
a sum of squares of ratios of absolute values of differences between the ECG signal of the first lead and the corresponding reconstructed signal to an unsigned greatest value is greater than a fourth threshold, wherein the unsigned greatest value is a greatest value in absolute values of amplitudes of the ECG signal of the first lead; or
a standard deviation of differences between the ECG signal of the first lead and the corresponding reconstructed signal is greater than a fifth threshold; or
a standard deviation of ratios of differences between the ECG signal of the first lead and the corresponding reconstructed signal to an unsigned greatest value is greater than a sixth threshold, wherein the unsigned greatest value is a greatest value in absolute values of amplitudes of the ECG signal of the first lead; or
an X1 percentile of absolute values of differences between the ECG signal of the first lead and the corresponding reconstructed signal is greater than a seventh threshold, wherein X1 is a preset value; or
an X2 percentile of ratios of absolute values of differences between the ECG signal of the first lead and the corresponding reconstructed signal to an unsigned greatest value is greater than an eighth threshold, wherein X2 is a preset value, and the unsigned greatest value is a greatest value in absolute values of amplitudes of the ECG signal of the first lead.

3. The method according to claim 1 or 2, wherein the determining the coronary heart disease probability of the user based on the abnormal signal and a corresponding reconstructed signal comprises:
determining a target signal based on the abnormal signal and the corresponding reconstructed signal, wherein the target signal reflects a difference between the abnormal signal and the corresponding reconstructed signal; and
determining the coronary heart disease probability of the user based on the target signal by using a third model, wherein
the third model is obtained through training based on a first signal set and a second signal set, wherein the first signal set comprises target signals respectively corresponding to ECG signals of leads of a patient having coronary heart disease, the target signals respectively corresponding to the ECG signals of the leads of the patient having coronary heart disease are determined by the ECG signals of the leads of the patient having coronary heart disease and reconstructed signals respectively corresponding to the ECG signals of the leads of the patient having coronary heart disease, the second signal set comprises target signals respectively corresponding to ECG signals of the leads of a healthy population, and the target signals respectively corresponding to the ECG signals of the leads of the healthy population are determined by the ECG signals of the leads of the healthy population and reconstructed signals respectively corresponding to the ECG signals of the leads of the healthy population.

4. The method according to claim 1 or 2, wherein the determining the coronary heart disease probability of the user based on the abnormal signal and a corresponding reconstructed signal comprises:
determining a target signal based on the abnormal signal and the corresponding reconstructed signal, wherein the target signal reflects a difference between the abnormal signal and the corresponding reconstructed signal; and
determining the coronary heart disease probability of the user based on the target signal by using a third model, wherein
the third model is obtained through training based on a first signal set, a second signal set, and a third signal set, wherein the first signal set comprises target signals respectively corresponding to ECG signals of leads of a patient having coronary heart disease, the target signals respectively corresponding to the ECG signals of the leads of the patient having coronary heart disease are determined by the ECG signals of the leads of the patient having coronary heart disease and reconstructed signals respectively corresponding to the ECG signals of the leads of the patient having coronary heart disease, the second signal set comprises target signals respectively corresponding to ECG signals of leads of a healthy population, the target signals respectively corresponding to the ECG signals of the leads of the healthy population are determined by the ECG signals of the leads of the healthy population and reconstructed signals respectively corresponding to the ECG signals of the leads of the healthy population, the third signal set comprises target signals respectively corresponding to ECG signals of leads of a population having another disease, and the target signals respectively corresponding to the ECG signals of the leads of the population having another disease are determined by the ECG signals of the leads of the population having another disease and reconstructed signals respectively corresponding to the ECG signals of the leads of the population having another disease.

5. The method according to claim 3 or 4, wherein the method further comprises:
determining a first feature based on the abnormal signal and the corresponding reconstructed signal by using the third model;
determining location information of the first feature in coronary heart disease lesion space based on the first feature; and
determining a most probable coronary heart disease lesion location of the user based on the location information of the first feature in the coronary heart disease lesion space.

6. The method according to claim 5, wherein the coronary heart disease lesion space is obtained through training based on the first signal set and diagnostic labels of the ECG signals of the leads of the patient having coronary heart disease.

7. The method according to claim 4 or 5, wherein the first feature is a fully-connected layer feature.

8. An apparatus (1200) for determining a coronary heart disease probability, wherein the apparatus (1200) comprises:
a transceiver unit (1220), configured to obtain pre-obtained ECG signals of one or more leads of a user; and
a processing unit (1210), configured to: determine, based on the ECG signals of the one or more leads, reconstructed signals respectively corresponding to the ECG signals of the one or more leads, wherein the ECG signals of the one or more leads comprise an ECG signal of a first lead, and a reconstructed signal corresponding to the ECG signal of the first lead is obtained by performing signal reconstruction based on a feature of the ECG signal of the first lead; and determine a coronary heart disease probability of the user based on the ECG signals of the one or more leads and the reconstructed signals respectively corresponding to the ECG signals of the one or more leads, wherein the processing unit (1210) is configured to: when the coronary heart disease probability of the user is determined based on the ECG signals of the one or more leads and the reconstructed signals respectively corresponding to the ECG signals of the one or more leads, determine an abnormal signal in the ECG signals of the one or more leads based on the ECG signals of the one or more leads and the reconstructed signals respectively corresponding to the ECG signals of the one or more leads; and determine the coronary heart disease probability of the user based on the abnormal signal and a corresponding reconstructed signal, wherein the processing unit (1210) is configured to: when the reconstructed signals respectively corresponding to the ECG signals of the one or more leads are determined based on the ECG signals of the one or more leads, extract a feature of an ECG signal of each lead in the ECG signals of the one or more leads by using a first model; and perform signal reconstruction based on the feature of the ECG signal of each lead by using a second model, to obtain a corresponding reconstructed signal, wherein
the first model and the second model are obtained through training based on an ECG signal of each lead of a healthy population.

9. The apparatus (1200) according to claim 8, wherein the processing unit (1210) is configured to: when the abnormal signal in the ECG signals of the one or more leads is determined based on the ECG signals of the one or more leads and the reconstructed signals respectively corresponding to the ECG signals of the one or more leads, determine that the ECG signal of the first lead is an abnormal signal if the ECG signal of the first lead and the corresponding reconstructed signal meet one or more of the following abnormal signal conditions:
in differences between the ECG signal of the first lead and the corresponding reconstructed signal, an absolute value of a difference is greater than a first threshold; or
in differences between the ECG signal of the first lead and the corresponding reconstructed signal, a ratio of an absolute value of a difference to an unsigned greatest value is greater than a second threshold, wherein the unsigned greatest value is a greatest value in absolute values of amplitudes of the ECG signal of the first lead; or
a sum of squares of absolute values of differences between the ECG signal of the first lead and the corresponding reconstructed signal is greater than a third threshold; or
a sum of squares of ratios of absolute values of differences between the ECG signal of the first lead and the corresponding reconstructed signal to an unsigned greatest value is greater than a fourth threshold, wherein the unsigned greatest value is a greatest value in absolute values of amplitudes of the ECG signal of the first lead; or
a standard deviation of differences between the ECG signal of the first lead and the corresponding reconstructed signal is greater than a fifth threshold; or
a standard deviation of ratios of differences between the ECG signal of the first lead and the corresponding reconstructed signal to an unsigned greatest value is greater than a sixth threshold, wherein the unsigned greatest value is a greatest value in absolute values of amplitudes of the ECG signal of the first lead; or
an X1 percentile of absolute values of differences between the ECG signal of the first lead and the corresponding reconstructed signal is greater than a seventh threshold, wherein X1 is a preset value; or
an X2 percentile of ratios of absolute values of differences between the ECG signal of the first lead and the corresponding reconstructed signal to an unsigned greatest value is greater than an eighth threshold, wherein X2 is a preset value, and the unsigned greatest value is a greatest value in absolute values of amplitudes of the ECG signal of the first lead.

10. The apparatus (1200) according to claim 8 or 9, wherein the processing unit (1210) is configured to: when the coronary heart disease probability of the user is determined based on the abnormal signal and the corresponding reconstructed signal, determine a target signal based on the abnormal signal and the corresponding reconstructed signal, wherein the target signal reflects a difference between the abnormal signal and the corresponding reconstructed signal; and determine the coronary heart disease probability of the user based on the target signal by using a third model, wherein the third model is obtained through training based on a first signal set and a second signal set, wherein the first signal set comprises target signals respectively corresponding to ECG signals of leads of a patient having coronary heart disease, the target signals respectively corresponding to the ECG signals of the leads of the patient having coronary heart disease are determined by the ECG signals of the leads of the patient having coronary heart disease and reconstructed signals respectively corresponding to the ECG signals of the leads of the patient having coronary heart disease, the second signal set comprises target signals respectively corresponding to ECG signals of leads of a healthy population, and the target signals respectively corresponding to the ECG signals of the leads of the healthy population are determined by the ECG signals of the leads of the healthy population and reconstructed signals respectively corresponding to the ECG signals of the leads of the healthy population.

11. The apparatus (1200) according to claim 8 or 9, wherein the processing unit (1210) is configured to: when the coronary heart disease probability of the user is determined based on the abnormal signal and the corresponding reconstructed signal, determine a target signal based on the abnormal signal and the corresponding reconstructed signal, wherein the target signal reflects a difference between the abnormal signal and the corresponding reconstructed signal; and determine the coronary heart disease probability of the user based on the target signal by using a third model, wherein
the third model is obtained through training based on a first signal set, a second signal set, and a third signal set, wherein the first signal set comprises target signals respectively corresponding to ECG signals of leads of a patient having coronary heart disease, the target signals respectively corresponding to the ECG signals of the leads of the patient having coronary heart disease are determined by the ECG signals of the leads of the patient having coronary heart disease and reconstructed signals respectively corresponding to the ECG signals of the leads of the patient having coronary heart disease, the second signal set comprises target signals respectively corresponding to ECG signals of leads of a healthy population, the target signals respectively corresponding to the ECG signals of the leads of the healthy population are determined by the ECG signals of the leads of the healthy population and reconstructed signals respectively corresponding to the ECG signals of the leads of the healthy population, the third signal set comprises target signals respectively corresponding to ECG signals of leads of a population having another disease, and the target signals respectively corresponding to the ECG signals of the leads of the population having another disease are determined by the ECG signals of the leads of the population having another disease and reconstructed signals respectively corresponding to the ECG signals of the leads of the population having another disease.

12. The apparatus (1200) according to claim 10 or 11, wherein the processing unit (1210) is configured to: determine a first feature based on the abnormal signal and the corresponding reconstructed signal by using the third model; determine location information of the first feature in coronary heart disease lesion space based on the first feature; and determine a most probable coronary heart disease lesion location of the user based on the location information of the first feature in the coronary heart disease lesion space.

13. The apparatus (1200) according to claim 12, wherein the coronary heart disease lesion space is obtained through training based on the first signal set and diagnostic labels of the ECG signals of the leads of the patient having coronary heart disease.

14. The apparatus (1200) according to claim 12 or 13, wherein the first feature is a fully-connected layer feature.

15. A computer-readable storage medium, wherein the storage medium stores a computer program or instructions, and when the computer program is executed or the instructions are executed by a communication apparatus, the method according to any one of claims 1 to 7 is implemented.

## Patentansprüche

1. Computer-implementiertes Verfahren zum Bestimmen einer Wahrscheinlichkeit einer koronaren Herzkrankheit, wobei das Verfahren Folgendes umfasst:
Erlangen (Schritt 1) von zuvor erlangten Elektrokardiogramm-Signalen, EKG-Signalen, von einer oder mehreren Ableitungen eines Benutzers;
Bestimmen (Schritt 2), basierend auf den EKG-Signalen der einen oder der mehreren Ableitungen, rekonstruierter Signale, die jeweils den EKG-Signalen der einen oder der mehreren Ableitungen entsprechen, wobei die EKG-Signale der einen oder der mehreren Ableitungen ein EKG-Signal einer ersten Ableitung umfassen und
ein rekonstruiertes Signal, das dem EKG-Signal der ersten Ableitung entspricht, durch Durchführen einer Signalrekonstruktion basierend auf einem Merkmal des EKG-Signals der ersten Ableitung erlangt wird; und
Bestimmen (Schritt 3) einer Wahrscheinlichkeit einer koronaren Herzkrankheit des Benutzers basierend auf den EKG-Signalen der einen oder der mehreren Ableitungen und den rekonstruierten Signalen, die jeweils den EKG-Signalen der einen oder der mehreren Ableitungen entsprechen, wobei das Bestimmen (Schritt 2), basierend auf den EKG-Signalen der einen oder der mehreren Ableitungen, rekonstruierter Signale, die jeweils den EKG-Signalen der einen oder der mehreren Ableitungen entsprechen, Folgendes umfasst:
Extrahieren eines Merkmals eines EKG-Signals jeder Ableitung in den EKG-Signalen der einen oder der mehreren Ableitungen unter Verwendung eines ersten Modells; und
Durchführen einer Signalrekonstruktion unter Verwendung eines zweiten Modells basierend auf dem Merkmal des EKG-Signals jeder Ableitung, um ein entsprechendes rekonstruiertes Signal zu erlangen, wobei
das erste Modell und das zweite Modell durch Training basierend auf einem EKG-Signal jeder Ableitung einer gesunden Population erlangt werden, wobei das Bestimmen (Schritt 3) einer Wahrscheinlichkeit einer koronaren Herzkrankheit des Benutzers basierend auf den EKG-Signalen der einen oder der mehreren Ableitungen und den rekonstruierten Signalen, die jeweils den EKG-Signalen der einen oder der mehreren Ableitungen entsprechen, Folgendes umfasst:
Bestimmen eines abnormalen Signals in den EKG-Signalen der einen oder der mehreren Ableitungen basierend auf den EKG-Signalen der einen oder der mehreren Ableitungen und den entsprechenden rekonstruierten Signalen; und
Bestimmen der Wahrscheinlichkeit einer koronaren Herzkrankheit des Benutzers basierend auf dem abnormalen Signal und einem entsprechenden rekonstruierten Signal.

2. Verfahren nach Anspruch 1, wobei das Bestimmen eines abnormalen Signals in den EKG-Signalen der einen oder der mehreren Ableitungen basierend auf den EKG-Signalen der einen oder der mehreren Ableitungen und den rekonstruierten Signalen, die jeweils den EKG-Signalen der einen oder der mehreren Ableitungen entsprechen, Folgendes umfasst:
Bestimmen, dass das EKG-Signal der ersten Ableitung ein abnormales Signal ist, wenn das EKG-Signal der ersten Ableitung und das entsprechende rekonstruierte Signal eine oder mehrere der folgenden abnormalen Signalbedingungen erfüllen:
bei Unterschieden zwischen dem EKG-Signal der ersten Ableitung und dem entsprechenden rekonstruierten Signal ist ein absoluter Wert eines Unterschieds größer als ein erster Schwellenwert; oder
bei Unterschieden zwischen dem EKG-Signal der ersten Ableitung und dem entsprechenden rekonstruierten Signal ist ein Verhältnis eines absoluten Wertes eines Unterschieds zu einem größten Wert ohne Vorzeichen größer als ein zweiter Schwellenwert, wobei der größte Wert ohne Vorzeichen ein größter Wert in absoluten Werten von Amplituden des EKG-Signals der ersten Ableitung **ist;** oder
eine Summe der Quadrate der absoluten Werte der Unterschiede zwischen dem EKG-Signal der ersten Ableitung und dem entsprechenden rekonstruierten Signal ist größer als ein dritter Schwellenwert; oder
eine Summe der Quadrate von Verhältnissen von absoluten Werten von Unterschieden zwischen dem EKG-Signal der ersten Ableitung und dem entsprechenden rekonstruierten Signal zu einem größten Wert ohne Vorzeichen ist größer als ein vierter Schwellenwert, wobei der größte Wert ohne Vorzeichen ein größter Wert in absoluten Werten von Amplituden des EKG-Signals der ersten Ableitung ist; oder
eine Standardabweichung der Unterschiede zwischen dem EKG-Signal der ersten Ableitung und dem entsprechenden rekonstruierten Signal ist größer als ein fünfter Schwellenwert; oder
eine Standardabweichung der Verhältnisse der Unterschiede zwischen dem EKG-Signal der ersten Ableitung und dem entsprechenden rekonstruierten Signal zu einem größten Wert ohne Vorzeichen ist größer als ein sechster Schwellenwert, wobei der größte Wert ohne Vorzeichen ein größter Wert in absoluten Werten von Amplituden des EKG-Signals der ersten Ableitung ist; oder
ein X1-Perzentil der absoluten Werte der Unterschiede zwischen dem EKG-Signal der ersten Ableitung und dem entsprechenden rekonstruierten Signal ist größer als ein siebter Schwellenwert, wobei X1 ein voreingestellter Wert ist; oder
ein X2-Perzentil von Verhältnissen von absoluten Werten von Unterschieden zwischen dem EKG-Signal der ersten Ableitung und dem entsprechenden rekonstruierten Signal zu einem größten Wert ohne Vorzeichen ist größer als ein achter Schwellenwert, wobei X2 ein voreingestellter Wert ist und der größte Wert ohne Vorzeichen ein größter Wert in absoluten Werten von Amplituden des EKG-Signals der ersten Ableitung ist.

3. Verfahren nach Anspruch 1 oder 2, wobei das Bestimmen der Wahrscheinlichkeit einer koronaren Herzkrankheit des Benutzers basierend auf dem abnormalen Signal und einem entsprechenden rekonstruierten Signal Folgendes umfasst:
Bestimmen eines Zielsignals basierend auf dem abnormalen Signal und dem entsprechenden rekonstruierten Signal, wobei das Zielsignal einen Unterschied zwischen dem abnormalen Signal und dem entsprechenden rekonstruierten Signal widerspiegelt; und
Bestimmen der Wahrscheinlichkeit einer koronaren Herzkrankheit des Benutzers basierend auf dem Zielsignal unter Verwendung eines dritten Modells, wobei
das dritte Modell durch Training basierend auf einem ersten Signalsatz und einem zweiten Signalsatz erlangt wird, wobei der erste Signalsatz Zielsignale umfasst, die jeweils EKG-Signalen von Ableitungen eines Patienten, der eine koronare Herzkrankheit hat, entsprechen, wobei die Zielsignale, die jeweils den EKG-Signalen der Ableitungen des Patienten, der eine koronare Herzkrankheit hat, entsprechen, durch die EKG-Signale der Ableitungen des Patienten, der eine koronare Herzkrankheit hat, und rekonstruierte Signale, die jeweils den EKG-Signalen der Ableitungen des Patienten, der eine koronare Herzkrankheit hat, entsprechen, bestimmt werden, wobei der zweite Signalsatz Zielsignale umfasst, die jeweils EKG-Signalen der Ableitungen einer gesunden Population entsprechen, und die Zielsignale, die jeweils den EKG-Signalen der Ableitungen der gesunden Population entsprechen, durch die EKG-Signale der Ableitungen der gesunden Population und rekonstruierte Signale, die jeweils den EKG-Signalen der Ableitungen der gesunden Population entsprechen, bestimmt werden.

4. Verfahren nach Anspruch 1 oder 2, wobei das Bestimmen der Wahrscheinlichkeit einer koronaren Herzkrankheit des Benutzers basierend auf dem abnormalen Signal und einem entsprechenden rekonstruierten Signal Folgendes umfasst:
Bestimmen eines Zielsignals basierend auf dem abnormalen Signal und dem entsprechenden rekonstruierten Signal, wobei das Zielsignal einen Unterschied zwischen dem abnormalen Signal und dem entsprechenden rekonstruierten Signal widerspiegelt; und
Bestimmen der Wahrscheinlichkeit einer koronaren Herzkrankheit des Benutzers basierend auf dem Zielsignal unter Verwendung eines dritten Modells, wobei
das dritte Modell durch Training basierend auf einem ersten Signalsatz, einem zweiten Signalsatz und einem drittem Signalsatz erlangt wird, wobei der erste Signalsatz Zielsignale umfasst, die jeweils EKG-Signalen von Ableitungen eines Patienten, der eine koronare Herzkrankheit hat, entsprechen, die Zielsignale, die jeweils den EKG-Signalen der Ableitungen des Patienten, der eine koronare Herzkrankheit hat, entsprechen, durch die EKG-Signale der Ableitungen des Patienten, der eine koronare Herzkrankheit hat, und rekonstruierte Signale, die jeweils den EKG-Signalen der Ableitungen des Patienten, der eine koronare Herzkrankheit hat, entsprechen, bestimmt werden, der zweite Signalsatz Zielsignale umfasst, die jeweils EKG-Signalen von Ableitungen einer gesunden Population entsprechen, die Zielsignale, die jeweils den EKG-Signalen der Ableitungen der gesunden Population entsprechen, durch die EKG-Signale der Ableitungen der gesunden Population und rekonstruierte Signale, die jeweils den EKG-Signalen der Ableitungen der gesunden Population entsprechen, bestimmt werden, der dritte Signalsatz Zielsignale umfasst, die jeweils den EKG-Signalen von Ableitungen einer Population, die eine andere Krankheit hat, entsprechen, und die Zielsignale, die jeweils den EKG-Signalen der Ableitungen der Population, die eine andere Krankheit hat, entsprechen, durch die EKG-Signale der Ableitungen der Population, die eine andere Krankheit hat, und die rekonstruierten Signale, die jeweils den EKG-Signalen der Ableitungen der Population, die eine andere Krankheit hat, entsprechen, bestimmt werden.

5. Verfahren nach Anspruch 3 oder 4, wobei das Verfahren ferner Folgendes umfasst:
Bestimmen eines ersten Merkmals basierend auf dem abnormalen Signal und dem entsprechenden rekonstruierten Signal unter Verwendung des dritten Modells;
Bestimmen von Positionsinformationen des ersten Merkmals in einem Läsionsraum der koronaren Herzkrankheit basierend auf dem ersten Merkmal; und
Bestimmen der wahrscheinlichsten Läsionsposition einer koronaren Herzkrankheit des Benutzers basierend auf den Positionsinformationen des ersten Merkmals im Läsionsraum der koronaren Herzkrankheit.

6. Verfahren nach Anspruch 5, wobei der Läsionsraum der koronaren Herzkrankheit durch Training basierend auf dem ersten Signalsatz und diagnostischen Kennungen der EKG-Signale der Ableitungen des Patienten, der eine koronare Herzkrankheit hat, erlangt wird.

7. Verfahren nach Anspruch 4 oder 5, wobei das erste Merkmal ein vollständig verbundenes Schichtmerkmal ist.

8. Vorrichtung (1200) zum Bestimmen einer Wahrscheinlichkeit einer koronaren Herzkrankheit, wobei die Vorrichtung (1200) Folgendes umfasst:
eine Sendeempfängereinheit (1220), die dazu konfiguriert ist, vorab erlangte EKG-Signale von einer oder mehreren Ableitungen eines Benutzers zu erlangen; und
eine Verarbeitungseinheit (1210), die zu Folgendem konfiguriert ist: Bestimmen, basierend auf den EKG-Signalen der einen oder der mehreren Ableitungen, von rekonstruierten Signalen, die jeweils den EKG-Signalen der einen oder der mehreren Ableitungen entsprechen, wobei die EKG-Signale der einen oder der mehreren Ableitungen ein EKG-Signal einer ersten Ableitung umfassen und ein rekonstruiertes Signal, das dem EKG-Signal der ersten Ableitung entspricht, durch Durchführen einer Signalrekonstruktion basierend auf einem Merkmal des EKG-Signals der ersten Ableitung erlangt wird; und Bestimmen einer Wahrscheinlichkeit einer koronaren Herzkrankheit des Benutzers basierend auf den EKG-Signalen der einen oder der mehreren Ableitungen und den rekonstruierten Signalen, die jeweils den EKG-Signalen der einen oder der mehreren Ableitungen entsprechen, wobei die Verarbeitungseinheit (1210) zu Folgendem konfiguriert ist: wenn die Wahrscheinlichkeit einer koronaren Herzkrankheit des Benutzers basierend auf den EKG-Signalen der einen oder der mehreren Ableitungen und den rekonstruierten Signalen, die jeweils den EKG-Signalen der einen oder der mehreren Ableitungen entsprechen, bestimmt wird, Bestimmen eines abnormalen Signals in den EKG-Signalen der einen oder der mehreren Ableitungen basierend auf den EKG-Signalen der einen oder der mehreren Ableitungen und den rekonstruierten Signalen, die jeweils den EKG-Signalen der einen oder der mehreren Ableitungen entsprechen; und Bestimmen der Wahrscheinlichkeit einer koronaren Herzkrankheit des Benutzers basierend auf dem abnormalen Signal und einem entsprechenden rekonstruierten Signal, wobei die Verarbeitungseinheit (1210) zu Folgendem konfiguriert ist: wenn die rekonstruierten Signale, die jeweils den EKG-Signalen der einen oder der mehreren Ableitungen entsprechen, basierend auf den EKG-Signalen der einen oder der mehreren Ableitungen bestimmt werden, Extrahieren eines Merkmals eines EKG-Signals jeder Ableitung in den EKG-Signalen der einen oder der mehreren Ableitungen unter Verwendung eines ersten Modells; und Durchführen einer Signalrekonstruktion basierend auf dem Merkmal des EKG-Signals jeder Ableitung unter Verwendung eines zweiten Modells, um ein entsprechendes rekonstruiertes Signal zu erlangen, wobei
das erste Modell und das zweite Modell durch Training basierend auf einem EKG-Signal jeder Ableitung einer gesunden Population erlangt werden.

9. Vorrichtung (1200) nach Anspruch 8, wobei die Verarbeitungseinheit (1210) zu Folgendem konfiguriert ist: wenn das abnormale Signal in den EKG-Signalen der einen oder der mehreren Ableitungen basierend auf den EKG-Signalen der einen oder der mehreren Ableitungen und den rekonstruierten Signalen, die den EKG-Signalen der einen oder der mehreren Ableitungen entsprechen, bestimmt wird, Bestimmen, dass das EKG-Signal der ersten Ableitung ein abnormales Signal ist, wenn das EKG-Signal der ersten Ableitung und das entsprechende rekonstruierte Signal eine oder mehrere der folgenden abnormalen Signalbedingungen erfüllen:
bei Unterschieden zwischen dem EKG-Signal der ersten Ableitung und dem entsprechenden rekonstruierten Signal ist ein absoluter Wert eines Unterschieds größer als ein erster Schwellenwert; oder
bei Unterschieden zwischen dem EKG-Signal der ersten Ableitung und dem entsprechenden rekonstruierten Signal ist ein Verhältnis eines absoluten Wertes eines Unterschieds zu einem größten Wert ohne Vorzeichen größer als ein zweiter Schwellenwert, wobei der größte Wert ohne Vorzeichen ein größter Wert in absoluten Werten von Amplituden des EKG-Signals der ersten Ableitung ist; oder
eine Summe der Quadrate der absoluten Werte der Unterschiede zwischen dem EKG-Signal der ersten Ableitung und dem entsprechenden rekonstruierten Signal ist größer als ein dritter Schwellenwert; oder
eine Summe der Quadrate von Verhältnissen von absoluten Werten von Unterschieden zwischen dem EKG-Signal der ersten Ableitung und dem entsprechenden rekonstruierten Signal zu einem größten Wert ohne Vorzeichen ist größer als ein vierter Schwellenwert, wobei der größte Wert ohne Vorzeichen ein größter Wert in absoluten Werten von Amplituden des EKG-Signals der ersten Ableitung ist; oder
eine Standardabweichung der Unterschiede zwischen dem EKG-Signal der ersten Ableitung und dem entsprechenden rekonstruierten Signal ist größer als ein fünfter Schwellenwert; oder
eine Standardabweichung der Verhältnisse der Unterschiede zwischen dem EKG-Signal der ersten Ableitung und dem entsprechenden rekonstruierten Signal zu einem größten Wert ohne Vorzeichen ist größer als ein sechster Schwellenwert, wobei der größte Wert ohne Vorzeichen ein größter Wert in absoluten Werten von Amplituden des EKG-Signals der ersten Ableitung ist; oder
ein X1-Perzentil der absoluten Werte der Unterschiede zwischen dem EKG-Signal der ersten Ableitung und dem entsprechenden rekonstruierten Signal ist größer als ein siebter Schwellenwert, wobei X1 ein voreingestellter Wert ist; oder
ein X2-Perzentil von Verhältnissen von absoluten Werten von Unterschieden zwischen dem EKG-Signal der ersten Ableitung und dem entsprechenden rekonstruierten Signal zu einem größten Wert ohne Vorzeichen ist größer als ein achter Schwellenwert, wobei X2 ein voreingestellter Wert ist und der größte Wert ohne Vorzeichen ein größter Wert in absoluten Werten von Amplituden des EKG-Signals der ersten Ableitung ist.

10. Vorrichtung (1200) nach Anspruch 8 oder 9, wobei die Verarbeitungseinheit (1210) zu Folgendem konfiguriert ist: wenn die Wahrscheinlichkeit einer koronaren Herzkrankheit des Benutzers basierend auf dem abnormalen Signal und dem entsprechenden rekonstruierten Signal bestimmt wird, Bestimmen eines Zielsignals basierend auf dem abnormalen Signal und dem entsprechenden rekonstruierten Signal, wobei das Zielsignal einen Unterschied zwischen dem abnormalen Signal und dem entsprechenden rekonstruierten Signal widerspiegelt; und Bestimmen der Wahrscheinlichkeit einer koronaren Herzkrankheit des Benutzers basierend auf dem Zielsignal unter Verwendung eines dritten Modells, wobei das dritte Modell durch Training basierend auf einem ersten Signalsatz und einem zweiten Signalsatz erlangt wird, wobei der erste Signalsatz Zielsignale umfasst, die jeweils EKG-Signalen von Ableitungen eines Patienten, der eine koronare Herzkrankheit hat, entsprechen, wobei die Zielsignale, die jeweils den EKG-Signalen der Ableitungen des Patienten, der eine koronare Herzkrankheit hat, entsprechen, durch die EKG-Signale der Ableitungen des Patienten, der eine koronare Herzkrankheit hat, und rekonstruierte Signale, die jeweils den EKG-Signalen der Ableitungen des Patienten, der eine koronare Herzkrankheit hat, entsprechen, bestimmt werden, wobei der zweite Signalsatz Zielsignale umfasst, die jeweils EKG-Signalen von Ableitungen einer gesunden Population entsprechen, und die Zielsignale, die jeweils den EKG-Signalen der Ableitungen der gesunden Population entsprechen, durch die EKG-Signale der Ableitungen der gesunden Population und rekonstruierte Signale, die jeweils den EKG-Signalen der Ableitungen der gesunden Population entsprechen, bestimmt werden.

11. Vorrichtung (1200) nach Anspruch 8 oder 9, wobei die Verarbeitungseinheit (1210) zu Folgendem konfiguriert ist: wenn die Wahrscheinlichkeit einer koronaren Herzkrankheit des Benutzers basierend auf dem abnormalen Signal und dem entsprechenden rekonstruierten Signal bestimmt wird, Bestimmen eines Zielsignals basierend auf dem abnormalen Signal und dem entsprechenden rekonstruierten Signal, wobei das Zielsignal einen Unterschied zwischen dem abnormalen Signal und dem entsprechenden rekonstruierten Signal widerspiegelt; und Bestimmen der Wahrscheinlichkeit einer koronaren Herzkrankheit des Benutzers basierend auf dem Zielsignal unter Verwendung eines dritten Modells, wobei
das dritte Modell durch Training basierend auf einem ersten Signalsatz, einem zweiten Signalsatz und einem drittem Signalsatz erlangt wird, wobei der erste Signalsatz Zielsignale umfasst, die jeweils EKG-Signalen von Ableitungen eines Patienten, der eine koronare Herzkrankheit hat, entsprechen, die Zielsignale, die jeweils den EKG-Signalen der Ableitungen des Patienten, der eine koronare Herzkrankheit hat, entsprechen, durch die EKG-Signale der Ableitungen des Patienten, der eine koronare Herzkrankheit hat, und rekonstruierte Signale, die jeweils den EKG-Signalen der Ableitungen des Patienten, der eine koronare Herzkrankheit hat, entsprechen, bestimmt werden, der zweite Signalsatz Zielsignale umfasst, die jeweils EKG-Signalen von Ableitungen einer gesunden Population entsprechen, die Zielsignale, die jeweils den EKG-Signalen der Ableitungen der gesunden Population entsprechen, durch die EKG-Signale der Ableitungen der gesunden Population und rekonstruierte Signale, die jeweils den EKG-Signalen der Ableitungen der gesunden Population entsprechen, bestimmt werden, der dritte Signalsatz Zielsignale umfasst, die jeweils den EKG-Signalen von Ableitungen einer Population, die eine andere Krankheit hat, entsprechen, und die Zielsignale, die jeweils den EKG-Signalen der Ableitungen der Population, die eine andere Krankheit hat, entsprechen, durch die EKG-Signale der Ableitungen der Population, die eine andere Krankheit hat, und die rekonstruierten Signale, die jeweils den EKG-Signalen der Ableitungen der Population, die eine andere Krankheit hat, entsprechen, bestimmt werden.

12. Vorrichtung (1200) nach Anspruch 10 oder 11, wobei die Verarbeitungseinheit (1210) zu Folgendem konfiguriert ist: Bestimmen eines ersten Merkmals basierend auf dem abnormalen Signal und dem entsprechenden rekonstruierten Signal unter Verwendung des dritten Modells; Bestimmen von Positionsinformationen des ersten Merkmals in einem Läsionsraum der koronaren Herzkrankheit basierend auf dem ersten Merkmal; und Bestimmen einer wahrscheinlichsten Läsionsposition der koronaren Herzkrankheit des Benutzers basierend auf den Positionsinformationen des ersten Merkmals in dem Läsionsraum der koronaren Herzkrankheit.

13. Vorrichtung (1200) nach Anspruch 12, wobei der Läsionsraum der koronaren Herzkrankheit durch Training basierend auf dem ersten Signalsatz und diagnostischen Kennungen der EKG-Signale der Ableitungen des Patienten, der eine koronare Herzkrankheit hat, erlangt wird.

14. Vorrichtung (1200) nach Anspruch 12 oder 13, wobei das erste Merkmal ein vollständig verbundenes Schichtmerkmal ist.

15. Computerlesbares Speichermedium, wobei das Speichermedium ein Computerprogramm oder Anweisungen speichert und, wenn das Computerprogramm oder die Anweisungen durch eine Kommunikationsvorrichtung ausgeführt werden, das Verfahren nach einem der Ansprüche 1 bis 7 umgesetzt wird.

## Revendications

1. Procédé mis en œuvre par ordinateur pour déterminer une probabilité de coronaropathie, dans lequel le procédé comprend :
l'obtention (étape 1) de signaux d'électrocardiogramme ECG pré-obtenus d'une ou de plusieurs dérivations d'un utilisateur ;
la détermination (étape 2), sur la base des signaux ECG d'une ou de plusieurs dérivations, de signaux reconstruits correspondant respectivement aux signaux ECG des une ou plusieurs dérivations, dans lequel les signaux ECG des une ou plusieurs dérivations comprennent un signal ECG d'une première dérivation, et un signal reconstruit correspondant au signal ECG de la première dérivation est obtenu en réalisant une reconstruction de signal sur la base d'une caractéristique du signal ECG de la première dérivation ; et
la détermination (étape 3) d'une probabilité de coronaropathie de l'utilisateur sur la base des signaux ECG des une ou plusieurs dérivations et des signaux reconstruits correspondant respectivement aux signaux ECG des une ou plusieurs dérivations, dans lequel la détermination (étape 2), sur la base des signaux ECG des une ou plusieurs dérivations, des signaux reconstruits correspondant respectivement aux signaux ECG des une ou plusieurs dérivations comprend :
l'extraction d'une caractéristique d'un signal ECG de chaque dérivation dans les signaux ECG des une ou plusieurs dérivations à l'aide d'un premier modèle ; et
la réalisation d'une reconstruction de signal à l'aide d'un deuxième modèle sur la base de la caractéristique du signal ECG de chaque dérivation, pour obtenir un signal reconstruit correspondant, dans lequel
le premier modèle et le deuxième modèle sont obtenus à travers un entraînement sur la base d'un signal ECG de chaque dérivation d'une population saine, dans lequel la détermination (étape 3) d'une probabilité de coronaropathie de l'utilisateur sur la base des signaux ECG des une ou plusieurs dérivations et des signaux reconstruits correspondant respectivement aux signaux ECG des une ou plusieurs dérivations comprend :
la détermination d'un signal anormal dans les signaux ECG des une ou plusieurs dérivations sur la base des signaux ECG des une ou plusieurs dérivations et des signaux reconstruits correspondants ; et
la détermination de la probabilité de coronaropathie de l'utilisateur sur la base du signal anormal et d'un signal reconstruit correspondant.

2. Procédé selon la revendication 1, dans lequel la détermination d'un signal anormal dans les signaux ECG des une ou plusieurs dérivations sur la base des signaux ECG des une ou plusieurs dérivations et des signaux reconstruits correspondant respectivement aux signaux ECG des une ou plusieurs dérivations comprend :
la détermination du fait que le signal ECG de la première dérivation est un signal anormal si le signal ECG de la première dérivation et le signal reconstruit correspondant répondent à l'une ou plusieurs des conditions de signal anormal suivantes :
dans les différences entre le signal ECG de la première dérivation et le signal reconstruit correspondant, une valeur absolue d'une différence est supérieure à un premier seuil ; ou
dans les différences entre le signal ECG de la première dérivation et le signal reconstruit correspondant, un rapport d'une valeur absolue d'une différence à une valeur maximale non signée est supérieur à un deuxième seuil, dans lequel la valeur maximale non signée est une valeur maximale en valeurs absolues d'amplitudes du signal ECG de la première dérivation ; ou
une somme des carrés de valeurs absolues de différences entre le signal ECG de la première dérivation et le signal reconstruit correspondant est supérieure à un troisième seuil ; ou
une somme des carrés de rapports de valeurs absolues de différences entre le signal ECG de la première dérivation et le signal reconstruit correspondant à une valeur maximale non signée est supérieure à un quatrième seuil, dans lequel la valeur maximale non signée est une valeur maximale en valeurs absolues d'amplitudes du signal ECG de la première dérivation ; ou
un écart type de différences entre le signal ECG de la première dérivation et le signal reconstruit correspondant est supérieur à un cinquième seuil ; ou
un écart type de rapports de différences entre le signal ECG de la première dérivation et le signal reconstruit correspondant à une valeur maximale non signée est supérieur à un sixième seuil, dans lequel la valeur maximale non signée est une valeur maximale en valeurs absolues d'amplitudes du signal ECG de la première dérivation ; ou
un percentile X1 de valeurs absolues de différences entre le signal ECG de la première dérivation et le signal reconstruit correspondant est supérieur à un septième seuil, dans lequel X1 est une valeur prédéfinie ; ou
un percentile X2 de rapports de valeurs absolues de différences entre le signal ECG de la première dérivation et le signal reconstruit correspondant à une valeur maximale non signée est supérieur à un huitième seuil, dans lequel X2 est une valeur prédéfinie, et la valeur maximale non signée est une valeur maximale en valeurs absolues d'amplitudes du signal ECG de la première dérivation.

3. Procédé selon la revendication 1 ou 2, dans lequel la détermination de la probabilité de coronaropathie de l'utilisateur sur la base du signal anormal et d'un signal reconstruit correspondant comprend :
la détermination d'un signal cible sur la base du signal anormal et du signal reconstruit correspondant, dans lequel le signal cible reflète une différence entre le signal anormal et le signal reconstruit correspondant ; et
la détermination de la probabilité de coronaropathie de l'utilisateur sur la base du signal cible à l'aide d'un troisième modèle, dans lequel
le troisième modèle est obtenu à travers un entraînement sur la base d'un premier ensemble de signaux et d'un deuxième ensemble de signaux, dans lequel le premier ensemble de signaux comprend des signaux cibles correspondant respectivement aux signaux ECG de dérivations d'un patient souffrant d'une coronaropathie, les signaux cibles correspondant respectivement aux signaux ECG des dérivations du patient souffrant d'une coronaropathie sont déterminés par les signaux ECG des dérivations du patient souffrant d'une coronaropathie et les signaux reconstruits correspondant respectivement aux signaux ECG des dérivations du patient souffrant d'une coronaropathie, le deuxième ensemble de signaux comprend des signaux cibles correspondant respectivement aux signaux ECG des dérivations d'une population saine, et les signaux cibles correspondant respectivement aux signaux ECG des dérivations de la population saine sont déterminés par les signaux ECG des dérivations de la population saine et les signaux reconstruits correspondant respectivement aux signaux ECG des dérivations de la population saine.

4. Procédé selon la revendication 1 ou 2, dans lequel la détermination de la probabilité de coronaropathie de l'utilisateur sur la base du signal anormal et d'un signal reconstruit correspondant comprend :
la détermination d'un signal cible sur la base du signal anormal et du signal reconstruit correspondant, dans lequel le signal cible reflète une différence entre le signal anormal et le signal reconstruit correspondant ; et
la détermination de la probabilité de coronaropathie de l'utilisateur sur la base du signal cible à l'aide d'un troisième modèle, dans lequel
le troisième modèle est obtenu à travers un entraînement sur la base d'un premier ensemble de signaux, d'un deuxième ensemble de signaux et d'un troisième ensemble de signaux, dans lequel premier ensemble de signaux comprend des signaux cibles correspondant respectivement aux signaux ECG de dérivations d'un patient souffrant d'une coronaropathie, les signaux cibles correspondant respectivement aux signaux ECG des dérivations du patient souffrant d'une coronaropathie sont déterminés par les signaux ECG des dérivations du patient souffrant d'une coronaropathie et les signaux reconstruits correspondant respectivement aux signaux ECG des dérivations du patient souffrant d'une coronaropathie, le deuxième ensemble de signaux comprend des signaux cibles correspondant respectivement aux signaux ECG de dérivations d'une population saine, les signaux cibles correspondant respectivement aux signaux ECG des dérivations de la population saine sont déterminés par les signaux ECG des dérivations de la population saine et les signaux reconstruits correspondant respectivement aux signaux ECG des dérivations de la population saine, le troisième ensemble de signaux comprend des signaux cibles correspondant respectivement aux signaux ECG de dérivations d'une population souffrant d'une autre maladie, et les signaux cibles correspondant respectivement aux signaux ECG des dérivations de la population souffrant d'une autre maladie sont déterminés par les signaux ECG des dérivations de la population souffrant d'une autre maladie et les signaux reconstruits correspondant respectivement aux signaux ECG des dérivations de la population souffrant d'une autre maladie.

5. Procédé selon la revendication 3 ou 4, dans lequel le procédé comprend également :
la détermination d'une première caractéristique sur la base du signal anormal et du signal reconstruit correspondant à l'aide du troisième modèle ;
la détermination d'informations d'emplacement de la première caractéristique dans l'espace de lésion de coronaropathie sur la base de la première caractéristique ; et
la détermination d'un emplacement de lésion de coronaropathie le plus probable de l'utilisateur sur la base des informations d'emplacement de la première caractéristique dans l'espace de lésion de coronaropathie.

6. Procédé selon la revendication 5, dans lequel l'espace de lésion de coronaropathie est obtenu à travers un entraînement sur la base du premier ensemble de signaux et d'étiquettes de diagnostic des signaux ECG des dérivations du patient souffrant de coronaropathie.

7. Procédé selon la revendication 4 ou 5, dans lequel la première caractéristique est une caractéristique de couche entièrement connectée.

8. Appareil (1200) pour déterminer une probabilité de coronaropathie, dans lequel l'appareil (1200) comprend :
une unité émettrice-réceptrice (1220), configurée pour obtenir des signaux ECG pré-obtenus d'une ou de plusieurs dérivations d'un utilisateur ; et
une unité de traitement (1210), configurée pour : déterminer, sur la base des signaux ECG des une ou plusieurs dérivations, des signaux reconstruits correspondant respectivement aux signaux ECG des une ou plusieurs dérivations, dans lequel les signaux ECG des une ou plusieurs dérivations comprennent un signal ECG d'une première dérivation, et un signal reconstruit correspondant au signal ECG de la première dérivation est obtenu en réalisant une reconstruction de signal sur la base d'une caractéristique du signal ECG de la première dérivation ; et déterminer une probabilité de coronaropathie de l'utilisateur sur la base des signaux ECG des une ou plusieurs dérivations et des signaux reconstruits correspondant respectivement aux signaux ECG des une ou plusieurs dérivations, dans lequel l'unité de traitement (1210) est configurée pour : lorsque la probabilité de coronaropathie de l'utilisateur est déterminée sur la base des signaux ECG des une ou plusieurs dérivations et des signaux reconstruits correspondant respectivement aux signaux ECG des une ou plusieurs dérivations, déterminer un signal anormal dans les signaux ECG des une ou plusieurs dérivations sur la base des signaux ECG des une ou plusieurs dérivations et des signaux reconstruits correspondant respectivement aux signaux ECG des une ou plusieurs dérivations ; et déterminer la probabilité de coronaropathie de l'utilisateur sur la base du signal anormal et d'un signal reconstruit correspondant, dans lequel l'unité de traitement (1210) est configurée pour : lorsque les signaux reconstruits correspondant respectivement aux signaux ECG des une ou plusieurs dérivations sont déterminés sur la base des signaux ECG des une ou plusieurs dérivations, extraire une caractéristique d'un signal ECG de chaque dérivation dans les signaux ECG des une ou plusieurs dérivations à l'aide d'un premier modèle ; et réaliser une reconstruction de signal sur la base de la caractéristique du signal ECG de chaque dérivation à l'aide d'un deuxième modèle, pour obtenir un signal reconstruit correspondant, dans lequel le premier modèle et le deuxième modèle sont obtenus à travers un entraînement sur la base d'un signal ECG de chaque dérivation d'une population saine.

9. Appareil (1200) selon la revendication 8, dans lequel l'unité de traitement (1210) est configurée pour : lorsque le signal anormal dans les signaux ECG des une ou plusieurs dérivations est déterminé sur la base des signaux ECG des une ou plusieurs dérivations et des signaux reconstruits correspondant respectivement aux signaux ECG des une ou plusieurs dérivations, déterminer le fait que le signal ECG de la première dérivation est un signal anormal si le signal ECG de la première dérivation et le signal reconstruit correspondant remplissent l'une ou plusieurs des conditions de signal anormal suivantes :
dans les différences entre le signal ECG de la première dérivation et le signal reconstruit correspondant, une valeur absolue d'une différence est supérieure à un premier seuil ; ou
dans les différences entre le signal ECG de la première dérivation et le signal reconstruit correspondant, un rapport d'une valeur absolue d'une différence à une valeur maximale non signée est supérieur à un deuxième seuil, dans lequel la valeur maximale non signée est une valeur maximale en valeurs absolues d'amplitudes du signal ECG de la première dérivation ; ou
une somme des carrés de valeurs absolues de différences entre le signal ECG de la première dérivation et le signal reconstruit correspondant est supérieure à un troisième seuil ; ou
une somme des carrés de rapports de valeurs absolues de différences entre le signal ECG de la première dérivation et le signal reconstruit correspondant à une valeur maximale non signée est supérieure à un quatrième seuil, dans lequel la valeur maximale non signée est une valeur maximale en valeurs absolues d'amplitudes du signal ECG de la première dérivation ; ou
un écart type de différences entre le signal ECG de la première dérivation et le signal reconstruit correspondant est supérieur à un cinquième seuil ; ou
un écart type de rapports de différences entre le signal ECG de la première dérivation et le signal reconstruit correspondant à une valeur maximale non signée est supérieur à un sixième seuil, dans lequel la valeur maximale non signée est une valeur maximale en valeurs absolues d'amplitudes du signal ECG de la première dérivation ; ou
un percentile X1 de valeurs absolues de différences entre le signal ECG de la première dérivation et le signal reconstruit correspondant est supérieur à un septième seuil, dans lequel X1 est une valeur prédéfinie ; ou
un percentile X2 de rapports de valeurs absolues de différences entre le signal ECG de la première dérivation et le signal reconstruit correspondant à une valeur maximale non signée est supérieur à un huitième seuil, dans lequel X2 est une valeur prédéfinie, et la valeur maximale non signée est une valeur maximale en valeurs absolues d'amplitudes du signal ECG de la première dérivation.

10. Appareil (1200) selon la revendication 8 ou 9, dans lequel l'unité de traitement (1210) est configurée pour : lorsque la probabilité de coronaropathie de l'utilisateur est déterminée sur la base du signal anormal et du signal reconstruit correspondant, déterminer un signal cible sur la base du signal anormal et du signal reconstruit correspondant, dans lequel le signal cible reflète une différence entre le signal anormal et le signal reconstruit correspondant ; et déterminer la probabilité de coronaropathie de l'utilisateur sur la base du signal cible à l'aide d'un troisième modèle, dans lequel le troisième modèle est obtenu à travers un entraînement sur la base d'un premier ensemble de signaux et d'un deuxième ensemble de signaux, dans lequel le premier ensemble de signaux comprend des signaux cibles correspondant respectivement aux signaux ECG de dérivations d'un patient souffrant d'une coronaropathie, les signaux cibles correspondant respectivement aux signaux ECG des dérivations du patient souffrant d'une coronaropathie sont déterminés par les signaux ECG des dérivations du patient souffrant d'une coronaropathie et les signaux reconstruits correspondant respectivement aux signaux ECG des dérivations du patient souffrant d'une coronaropathie, le deuxième ensemble de signaux comprend des signaux cibles correspondant respectivement aux signaux ECG de dérivations d'une population saine, et les signaux cibles correspondant respectivement aux signaux ECG des dérivations de la population saine sont déterminés par les signaux ECG des dérivations de la population saine et les signaux reconstruits correspondant respectivement aux signaux ECG des dérivations de la population saine.

11. Appareil (1200) selon la revendication 8 ou 9, dans lequel l'unité de traitement (1210) est configurée pour : lorsque la probabilité de coronaropathie de l'utilisateur est déterminée sur la base du signal anormal et du signal reconstruit correspondant, déterminer un signal cible sur la base du signal anormal et du signal reconstruit correspondant, dans lequel le signal cible reflète une différence entre le signal anormal et le signal reconstruit correspondant ; et déterminer la probabilité de coronaropathie de l'utilisateur sur la base du signal cible à l'aide d'un troisième modèle, dans lequel
le troisième modèle est obtenu à travers un entraînement sur la base d'un premier ensemble de signaux, d'un deuxième ensemble de signaux et d'un troisième ensemble de signaux, dans lequel premier ensemble de signaux comprend des signaux cibles correspondant respectivement aux signaux ECG de dérivations d'un patient souffrant d'une coronaropathie, les signaux cibles correspondant respectivement aux signaux ECG des dérivations du patient souffrant d'une coronaropathie sont déterminés par les signaux ECG des dérivations du patient souffrant d'une coronaropathie et les signaux reconstruits correspondant respectivement aux signaux ECG des dérivations du patient souffrant d'une coronaropathie, le deuxième ensemble de signaux comprend des signaux cibles correspondant respectivement aux signaux ECG de dérivations d'une population saine, les signaux cibles correspondant respectivement aux signaux ECG des dérivations de la population saine sont déterminés par les signaux ECG des dérivations de la population saine et les signaux reconstruits correspondant respectivement aux signaux ECG des dérivations de la population saine, le troisième ensemble de signaux comprend des signaux cibles correspondant respectivement aux signaux ECG de dérivations d'une population souffrant d'une autre maladie, et les signaux cibles correspondant respectivement aux signaux ECG des dérivations de la population souffrant d'une autre maladie sont déterminés par les signaux ECG des dérivations de la population souffrant d'une autre maladie et les signaux reconstruits correspondant respectivement aux signaux ECG des dérivations de la population souffrant d'une autre maladie.

12. Appareil (1200) selon la revendication 10 ou 11, dans lequel l'unité de traitement (1210) est configurée pour : déterminer une première caractéristique sur la base du signal anormal et du signal reconstruit correspondant à l'aide du troisième modèle ; déterminer des informations d'emplacement de la première caractéristique dans l'espace de lésion de coronaropathie sur la base de la première caractéristique ; et déterminer un emplacement de lésion de coronaropathie le plus probable de l'utilisateur sur la base des informations d'emplacement de la première caractéristique dans l'espace de lésion de coronaropathie.

13. Appareil (1200) selon la revendication 12, dans lequel l'espace de lésion de coronaropathie est obtenu à travers un entraînement sur la base du premier ensemble de signaux et d'étiquettes de diagnostic des signaux ECG des dérivations du patient souffrant de coronaropathie.

14. Appareil (1200) selon la revendication 12 ou 13, dans lequel la première caractéristique est une caractéristique de couche entièrement connectée.

15. Support de stockage lisible par ordinateur, dans lequel le support de stockage stocke un programme informatique ou des instructions, et lorsque le programme informatique est exécuté ou que les instructions sont exécutées par un appareil de communication, le procédé selon l'une quelconque des revendications 1 à 7 est mis en œuvre.
